(19) Europäisches Patentamt
European Patent Office
Office européen des brevets

(11) Veröffentlichungsnummer: **0 415 211 B1**

(12) **EUROPÄISCHE PATENTSCHRIFT**

(45) Veröffentlichungstag der Patentschrift: **21.12.94**

(51) Int. Cl.[5]: **C07D 513/04**, A01N 43/90, C07D 279/06, C07D 279/10, C07D 277/06, //(C07D513/04, 279:00,209:00),(C07D513/04, 277:00,209:00)

(21) Anmeldenummer: **90115872.5**

(22) Anmeldetag: **18.08.90**

Die Akte enthält technische Angaben, die nach dem Eingang der Anmeldung eingereicht wurden und die nicht in dieser Patentschrift enthalten sind.

# (54) 3-Aryl-pyrrolidin-2,4-dion-derivate.

(30) Priorität: **01.09.89 DE 3929087**

(43) Veröffentlichungstag der Anmeldung:
**06.03.91 Patentblatt 91/10**

(45) Bekanntmachung des Hinweises auf die Patenterteilung:
**21.12.94 Patentblatt 94/51**

(84) Benannte Vertragsstaaten:
**BE CH DE DK FR GB IT LI NL**

(56) Entgegenhaltungen:
**EP-A- 0 090 312**
**EP-A- 0 262 399**
**DE-A- 2 361 084**

(73) Patentinhaber: **BAYER AG**

**D-51368 Leverkusen (DE)**

(72) Erfinder: **Fischer, Reiner, Dr.**
**Nelly-Sachs-Strasse 23**
**D-4019 Monheim 2 (DE)**
Erfinder: **Hagemann, Hermann, Dr.**
**Kandinsky-Strasse 52**
**D-5090 Leverkusen 1 (DE)**
Erfinder: **Krebs, Andreas, Dr.**
**Im Gartenfeld 70**
**D-5068 Odenthal-Holz (DE)**
Erfinder: **Marhold, Albrecht, Dr.**
**Carl-Duisberg-Strasse 329**
**D-5090 Leverkusen 1 (DE)**
Erfinder: **Santel, Hans-Joachim, Dr.**
**Grünstrasse 9a**
**D-5090 Leverkusen 1 (DE)**
Erfinder: **Schmidt, Robert-R., Dr.**
**Im Waldwinkel 110**
**D-5060 Bergisch Gladbach 2 (DE)**
Erfinder: **Lürssen, Klaus, Dr.**
**August-Kierspel-Strasse 145**
**D-5060 Bergisch Gladbach 2 (DE)**
Erfinder: **Becker, Benedikt, Dr.**
**Via Max Sparer 17**
**I-39057 Appiano (BZ) (IT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **Stendel, Wilhelm, Dr.**
**In den Birken 55**
**D-5600 Wuppertal 1 (DE)**
Erfinder: **Erdelen, Christoph, Dr.**
**Unterbüscherhof 22**
**D-5653 Leichlingen 1 (DE)**

## Beschreibung

Die Erfindung betrifft neue 3-Aryl-pyrrolidin-2,4-dion-Derivate, mehrere Verfahren zu ihrer Herstellung und ihre Verwendung als Herbizide, Insektizide und Akarizide.

Von 3-Acyl-pyrrolidin-2,4-dionen sind pharmazeutische Eigenschaften vorbeschrieben (S. Suzuki et. al. Chem. Pharm. bull. 15 1120 (1967)). Weiterhin wurden N-Phenyl-pyrrolidin-2,4-dione von R. Schmierer und H. Mildenberger Liebigs Ann. Chem. 1985 1095 synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine herbizide, fungizide, antimykotische, tickizide, insektizide oder akarizide Wirksamkeit bekannt geworden ist.

Aus der DE-A-2 361 084 sind substituierte 3-Hydroxyindone mit herbizider Wirkung bekannt. Die Wirksamkeit und die Wirkungsbreite dieser Verbindungen ist jedoch nicht immer zufriedenstellend.

Es wurden nun neue 3-Aryl-pyrrolidin-2,4-dion-Derivate gefunden, die durch die Formel (I) dargestellt sind,

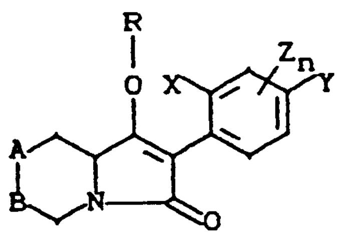

(I)

in welcher

A-B für $-S-CH_2-$, $-SO-CH_2-$, $-SO_2-CH_2-$, $-CH_2-S-$, $-CH_2-SO-$, $-CH_2-SO_2-$, -S-, -SO-, $-SO_2-$ steht,

X für $C_1$-$C_6$-Alkyl, Halogen, $C_1$-$C_6$-Alkoxy steht,

Y für Wasserstoff, $C_1$-$C_6$-Alkyl, Halogen, $C_1$-$C_6$-Alkoxy, $C_1$-$C_3$-Halogenalkyl steht,

Z für $C_1$-$C_6$-Alkyl, Halogen, $C_1$-$C_6$-Alkoxy steht,

n für eine Zahl von 0-3 steht,

R für Wasserstoff (Ia) oder für die Gruppen der Formel

$-CO-R^1$ (Ib), $-CO-O-R^2$ (Ic)

oder E (Id)

steht, in welchen

E für ein Metallkationäquivalent oder für ein Ammoniumion steht,

$R^1$ für gegebenenfalls durch Halogen substituiertes $C_1$-$C_{20}$-Alkyl, $C_2$-$C_{20}$-Alkenyl, $C_1$-$C_8$-Alkoxy-$C_1$-$C_8$-alkyl, $C_1$-$C_8$-Alkylthio-$C_1$-$C_8$-alkyl, $C_1$-$C_8$-Polyalkoxy-$C_2$-$C_8$-alkyl und Cycloalkyl mit 3-8 Ringatomen, das durch Sauerstoff und/oder Schwefel unterbrochen sein kann, steht,

für gegebenenfalls durch Halogen, Nitro, $C_1$-$C_6$-Alkyl, $C_1$-$C_6$-Alkoxy, $C_1$-$C_6$-Halogenalkyl, $C_1$-$C_6$-Halogenalkoxy substituiertes Phenyl;

für gegebenenfalls durch Halogen, $C_1$-$C_6$-Alkyl, $C_1$-$C_6$-Alkoxy, $C_1$-$C_6$-Halogenalkyl, $C_1$-$C_6$-Halogenalkoxy substituierteS Phenyl-$C_1$-$C_6$-alkyl steht,

für gegebenenfalls durch Halogen und $C_1$-$C_6$-Alkyl-substituiertes Pyridyl, Pyrimidyl, Thiazolyl und Pyrazolyl steht,

für gegebenenfalls durch Halogen und $C_1$-$C_6$-Alkyl substituiertes Phenoxy-$C_1$-$C_6$-alkyl steht,

für gegebenenfalls durch Halogen, Amino und $C_1$-$C_6$-Alkyl substituiertes Pyridyloxy-$C_1$-$C_4$-alkyl, Pyrimidyloxy-$C_1$-$C_4$-alkyl und Thiazolyloxy-$C_1$-$C_4$-alkyl steht,

$R^2$ für gegebenenfalls durch Halogen substituiiertes $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Polyalkoxy-$C_2$-$C_8$-alkyl steht,

für gegebenenfalls durch Halogen, Nitro, $C_1$-$C_6$-Alkyl, $C_1$-$C_6$-Alkoxy, $C_1$-$C_6$-Halogenalkyl substituiertes Phenyl steht,

sowie die enantiomerenreinen Formen von Verbindungen der Formel (I).

Im folgenden seien die folgenden Untergruppen definiert:

(Ia): Verbindungen der Formel (I), worin R = Wasserstoff,
(Ib): Verbindungen der Formel (I), worin R = $COR^1$,
(Ic): Verbindungen der Formel (I), worin R = $COOR^2$,
(Id): Verbindungen der Formel (I), worin R = ein Metallionäquivalent oder ein Ammoniumion.
(Ie): Verbindungen der Formel (I), worin A-B = $-SO-CH_2-$, $-CH_2-SO$ oder -SO-,
(If): Verbindungen der Formel (I), worin A-B = $-SO_2-CH_2-$, $-CH_2-SO_2-$, $-SO_2-$ darstellt.

Weiterhin wurde gefunden, daß man 3-Aryl-pyrrolidin-2,4-dione bzw. deren Enole der Formel (Ia)

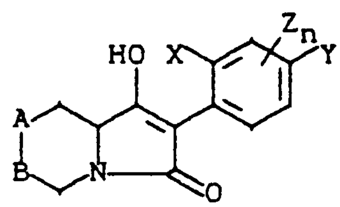

(Ia)

erhält, wenn man

(A)

N-Acylaminosäureester der Formel (II),

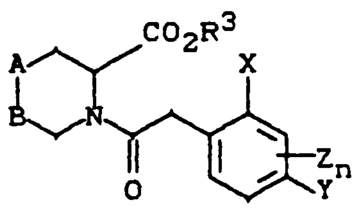

(II)

in welcher

A, B, X, Y, Z und n die oben angegebene Bedeutung haben

und

$R^3$ für Alkyl steht,

in Gegenwart eines Verdünnungsmittels und in Gegenwart einer Base intramolekular kondensiert.

(B)

Außerdem wurde gefunden, daß man Verbindungen der Formel (Ib)

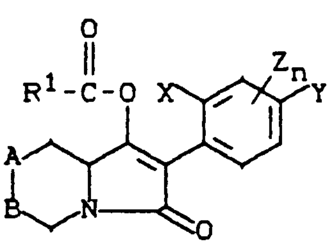

(Ib)

erhält, wenn man Verbindungen der Formel (Ia),

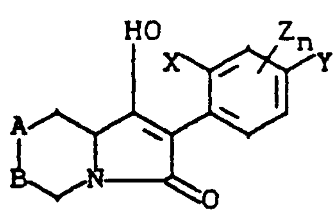

(Ia)

in welcher

A, B, X, Y, Z und n die oben angegebene Bedeutung haben,

α) mit Säurehalogeniden der allgemeinen Formel (III),

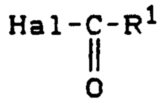

(III)

in welcher

$R^1$ die oben angegebene Bedeutung hat

und

Hal für Halogen, insbesondere Chlor und Brom steht,

gegebenenfalls in Gegenwart eines Verdünnungsmittels und gegebenenfalls in Gegenwart eines Säurebindemittels,

oder

β) mit Carbonsäureanhydriden der allgemeinen Formel (IV),

$R^1$-CO-O-CO-$R^1$ (IV)

in welcher

$R^1$ die oben angegebene Bedeutung hat,

gegebenenfalls in Gegenwart eines Verdünnungsmittels und gegebenenfalls in Gegenwart eines Säurebindemittels,

umsetzt.

(C)

Ferner wurde gefunden, daß man Verbindungen der Formel (Ic)

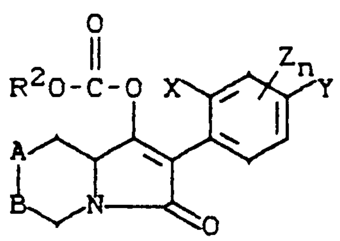

(Ic)

erhält, wenn man Verbindungen der Formel (Ia),

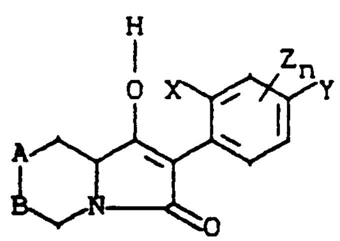

(Ia)

in welcher

A, B, X, Y, Z und n die oben angegebene Bedeutung haben

mit Chlorameisensäureester der allgemeinen Formel (V),

$R^2$-O-CO-Cl (V)

in welcher

$R^2$ die oben angegebene Bedeutung hat,

gegebenenfalls in Gegenwart eines Verdünnungsmittels und gegebenenfalls in Gegenwart eines Säurebindemittels umsetzt.

(D)

Weiterhin wurde gefunden, daß man Verbindungen der Formel (Id),

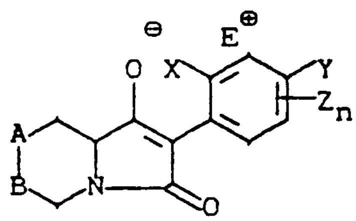

(Id)

in welcher

A, B, X, Y, Z, E und n die oben angegebene Bedeutung haben,

erhält, wenn man Verbindungen der Formel (Ia),

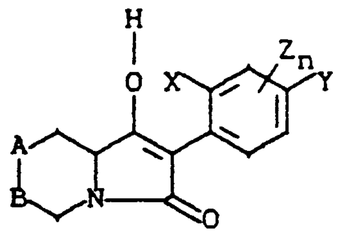

(Ia)

in welcher

A, B, X, Y, Z und n die oben angegebene Bedeutung haben,

mit Metallhydroxiden oder Aminen der allgemeinen Formeln (VIII) und (IX),

$Me_sOH_t$ (VIII)

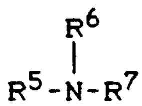

(IX)

in welchen

| | |
|---|---|
| Me | für ein- oder zweiwertige Metallionen |
| s und t | für die Zahl 1 und 2 und |
| $R^5$, $R^6$ und $R^7$ | unabhängig voneinander für Wasserstoff und Alkyl |

stehen,

gegebenenfalls in Gegenwart eines Verdünnungsmittels, umsetzt.

(E)

Ferner wurde gefunden, daß man Verbindungen der Formel (Ie),

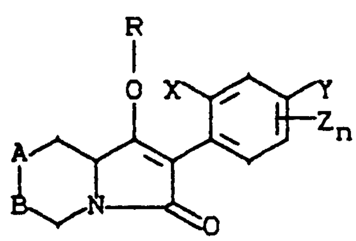

(Ie)

in welcher

A, B, R, X, Y, Z und n die oben angegebene Bedeutung haben,

erhält, wenn man Verbindungen der Formel (Ia)-(Ic),

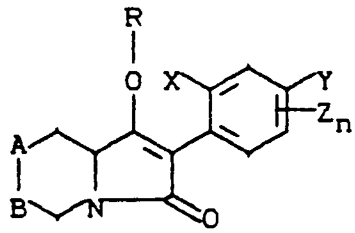

(Ia)-(Ic)

in welcher

R, X, Y, Z und n die oben angegebene Bedeutung haben

und

A-B für $-S-CH_2-$, $-CH_2-S-$ oder -S- steht,

mit annähernd äquimolaren Mengen eines Oxidationsmittels, gegebenenfalls in Gegenwart eines Verdünnungsmittels, umsetzt

(F)

Außerdem wurde gefunden, daß man Verbindungen der Formel (If),

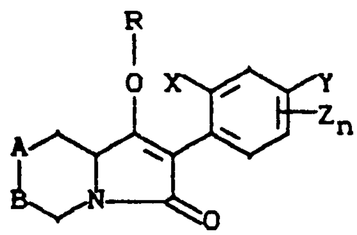

(If)

in welcher

A, B, R, X, Y, Z und n die oben angegebene Bedeutung haben,

erhält, wenn man Verbindungen der Formel (Ia)-(Ic),

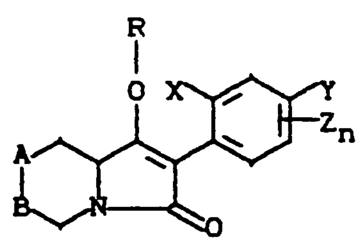

(Ia)-(Ic)

in welcher

R, X, Y, Z und n die oben angegebene Bedeutung haben

und

A-B für $-S-CH_2-$, $-CH_2-S-$ oder -S-

mit doppelt äquimolaren Mengen eines Oxidationsmittels, gegebenenfalls in Gegenwart eines Verdünnungsmittels, umsetzt.

Überraschenderweise wurde gefunden, daß die neuen 3-Arylpyrrolidin-2,4-dione der Formel (I) sich durch hervorragende herbizide, insektizide, antimykotische und akarizide Wirkungen auszeichnen.

Bevorzugt sind kondensierte 1,5-Alkylen-3-aryl-pyrrolidin-2,4-dione und deren entsprechende Enolester der Formel (I), in welcher

A-B für $-S-CH_2-$, $-SO-CH_2-$, $-SO_2-CH_2-$, $-CH_2-S-$, $-CH_2-SO-$, $-CH_2-SO_2-$, -S-, -SO-, $-SO_2-$ steht,

X für $C_1$-$C_4$-Alkyl, Halogen, $C_1$-$C_4$-Alkoxy steht,
Y für Wasserstoff, $C_1$-$C_6$-Alkyl, Halogen, $C_1$-$C_4$-Alkoxy, $C_1$-$C_2$-Halogenalkyl steht,
Z für $C_1$-$C_4$-Alkyl, Halogen, $C_1$-$C_4$-Alkoxy steht,
n für eine Zahl von 0-3 steht,
R für Wasserstoff (Ia) oder für die Gruppen der Formel

-CO-$R^1$ (Ib), -CO-O-$R^2$ (Ic)

oder E (Id)

steht, in welchen
E für ein Metallkationäquivalent oder für ein Ammoniumion steht,
$R^1$ für gegebenenfalls durch Halogen substituiertes $C_1$-$C_{16}$-Alkyl, $C_2$-$C_{16}$-Alkenyl, $C_1$-$C_6$-Alkoxy-$C_1$-$C_6$-alkyl, $C_1$-$C_6$-Alkylthio-$C_1$-$C_6$-alkyl, $C_1$-$C_6$-Polyalkoxy-$C_2$-$C_6$-alkyl und Cycloalkyl mit 3-7 Ringatomen, das durch 1-2 Sauerstoff- und/oder Schwefelatome unterbrochen sein kann steht,
für gegebenenfalls durch Halogen, Nitro, $C_1$-$C_4$-Alkyl, $C_1$-$C_4$-Alkoxy, $C_1$-$C_3$-Halogenalkyl, $C_1$-$C_3$-Halogenalkoxy substituiertes Phenyl steht,
für gegebenenfalls durch Halogen, $C_1$-$C_4$-Alkyl, $C_1$-$C_4$-Alkoxy, $C_1$-$C_3$-Halogenalkyl, $C_1$-$C_3$-Halogenalkoxy substituiertes Phenyl-$C_1$-$C_4$-alkyl steht,
für gegebenenfalls duch Halogen und $C_1$-$C_6$-Alkyl-substituiertes Pyridyl, Pyrimidyl, Thiazolyl und Pyrazolyl steht,
gegebenenfalls für durch Halogen und $C_1$-$C_4$-Alkyl substituiertes Phenoxy-$C_1$-$C_5$-alkyl steht,
für gegebenenfalls durch Halogen, Amino und $C_1$-$C_4$-Alkyl substituiertes Pyridyloxy-$C_1$-$C_4$-alkyl, Pyrimidyloxy-$C_1$-$C_4$-alkyl und Thiazolyloxy-$C_1$-$C_4$-alkyl steht,
$R^2$ für gegebenenfalls durch Halogen substituiertes $C_1$-$C_{16}$-Alkyl, $C_2$-$C_{16}$-Alkenyl, $C_1$-$C_{16}$-Alkoxy-$C_2$-$C_6$-alkyl, $C_1$-$C_6$-Polyalkoxy-$C_2$-$C_6$-alkyl steht,
für gegebenenfalls durch Halogen, Nitro, $C_1$-$C_4$-Alkyl, $C_1$-$C_3$-Alkoxy, $C_1$-$C_3$-Halogenalkyl-substituiertes Phenyl steht,

sowie die enantiomerenreinen Formen von Verbindungen der Formel (I).

Besonders bevorzugt sind Verbindungen der Formel (I), in welcher
A-B für -S-$CH_2$-, -SO-$CH_2$-, -$SO_2$-$CH_2$-, -$CH_2$-S-, -$CH_2$-SO-, -$CH_2SO_2$-, -S-, -SO-, -$SO_2$ steht
X für Methyl, Ethyl, Propyl, i-Propyl, Fluor, Chlor, Brom, Methoxy und Ethoxy steht,
Y für Wasserstoff, Methyl, Ethyl, Propyl, i-Propyl, Butyl, i-Butyl, tert.-Butyl, Chlor, Brom, Methoxy, Ethoxy und Trifluormethyl steht,
Z für Methyl, Ethyl, i-Propyl, Butyl, i-Butyl, tert.-Butyl, Chlor, Brom, Methoxy und Ethoxy steht,
n für eine Zahl von 0-3 steht,
R für Wasserstoff (Ia) oder für die Gruppen der Formel

-CO-$R^1$ (Ib), -CO-O-$R^2$ (Ic)

oder E (Id)

steht, in welcher
E für ein Metallkationäquivalent oder für ein Ammoniumion steht,
$R^1$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_1$-$C_6$-alkyl, $C_1$-$C_4$-Alkylthio-$C_1$-$C_6$-alkyl, $C_1$-$C_4$-Polyalkoxyl-$C_2$-$C_4$-alkyl und Cycloalkyl mit 3-6 Ringatomen, das durch 1-2 Sauerstoff- und/oder Schwefelatome unterbrochen sein kann steht,
für gegebenenfalls durch Fluor, Chlor, Brom, Methyl, Ethyl, Propyl, i-Propyl, Methoxy, Ethoxy, Trifluormethyl-, Trifluormethoxy, Nitro substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-$C_1$-$C_3$-alkyl steht,
für gegebenenfalls durch Fluor, Chlor, Brom, Methyl, Ethyl substituiertes Pyridyl, Pyrimidyl, Thiazolyl und Pyrazolyl steht,
für gegebenenfalls durch Fluor, Chlor, Methyl, Ethyl substituiertes Phenoxy-$C_1$-$C_4$-alkyl steht,
für gegebenenfalls durch Fluor, Chlor, Amino, Methyl, Ethyl, substituiertes Pyridyloxy-$C_1$-$C_4$-alkyl, Pyrimidyloxy-$C_1$-$C_4$-alkyl und Thiazolyloxy-$C_1$-$C_4$-alkyl steht,

$R^2$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Polyalkoxy-$C_2$-$C_6$-alkyl steht,
für gegebenenfalls durch Fluor, Chlor, Nitro, Methyl, Ethyl, Propyl, i-Propyl, Methoxy, Ethoxy, Trifluormethyl, substituiertes Phenyl steht,

sowie die enantiomerenreinen Formen von Verbindungen der Formel I.

Verwendet man gemäß Verfahren (A) N-(2,6-Dichlorphenylacetyl)-1,4-thiomorpholin-3-carbonsäureethylester, so kann der Verlauf des erfindungsgemäßen Verfahrens durch folgendes Reaktionsschema wiedergegeben werden:

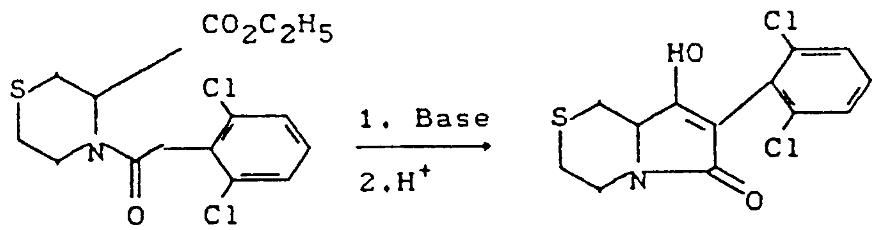


Verwendet man gemäß Verfahren (B) (Variante $\alpha$) 3-(2,4,6-Trimethylphenyl)-1,5-ethylmercaptomethyl-pyrrolidin-2,4-dion und Pivaloylchlorid als Ausgangsstoff, so kann der Verlauf des erfindungsgemäßen Verfahrens durch folgendes Reaktionsschema wiedergegeben werden.

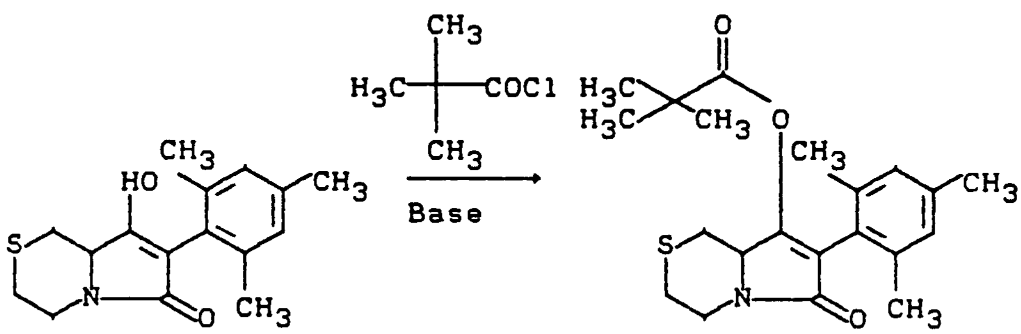


Verwendet man gemäß Verfahren B (Variante $\beta$) 3-(2,4,5-Trimethylphenyl)-1,5-methylmercaptoethyl-pyrrolidin-2,4-dion und Acetanhydrid, so kann der Verlauf des erfindungsgemäßen Verfahrens durch folgendes Reaktionsschema wiedergegeben werden.

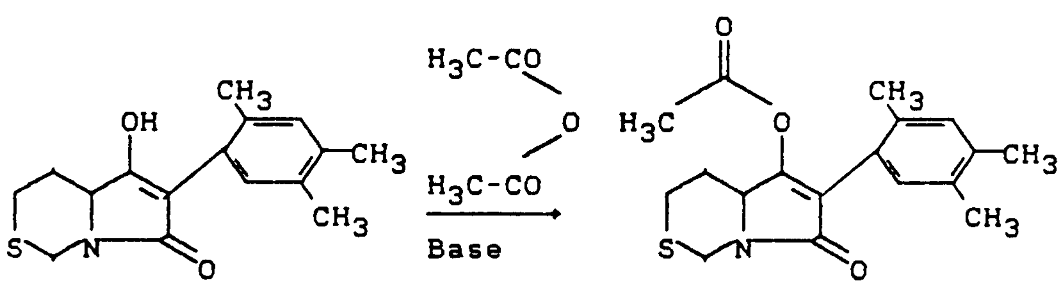


Verwendet man gemäß Verfahren (C) 3-(2,4-Dichlorphenyl)-1,5-methylmercaptoethyl-pyrrolidin-2,4-dion und Chlorameisensäureethoxyethylester, so kann der Verlauf des erfindungsgemäßen Verfahrens durch folgendes Reaktionsschema wiedergegeben werden.

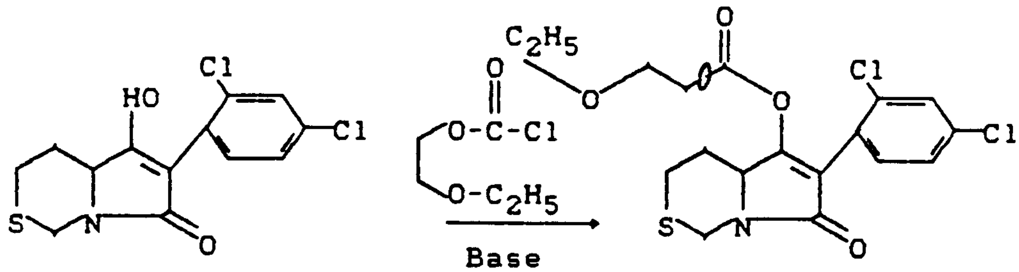


Verwendet man gemäß Verfahren (D) 3-(2,4,6-Trimethylphenyl-1,5-ethylmercaptomethyl-pyrrolidin-2,4-dion und NaOH, so kann der Verlauf des erfindungsgemäßen Verfahrens durch folgendes Reaktionsschema wiedergegeben werden:

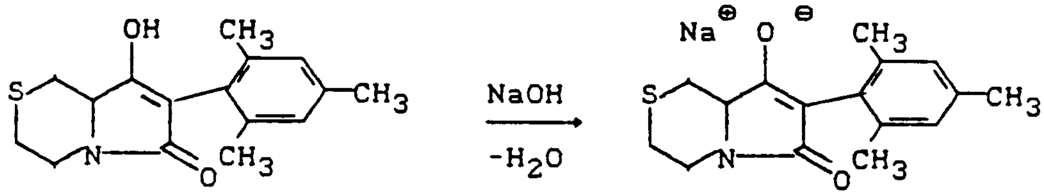


Verwendet man gemäß Verfahren (E) 3-(2,6-Dichlorphenyl)-1,5-ethylmercaptomethyl-pyrrolidin-2,4-dion und äquimolare Mengen an m-Chlorperbenzoesäure, so kann der Verlauf des erfindungsgemäßen Verfahren durch folgendes Reaktionsschema wiedergegeben werden:

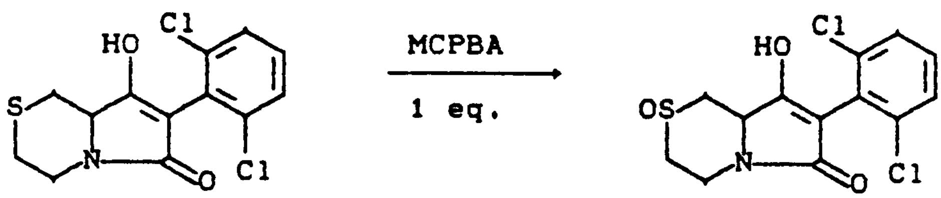


Verwendet man gemäß Verfahren (F) 4-(Pivaloyloxy)-3-2,4,6-trimethylphenyl)-1,5-ethylmercaptomethyl-3-pyrrolin-2-on und doppelt äquimolare Mengen m-Chlorperbenzoesäure, so kann der Verlauf des erfindungsgemäßen Verfahrens durch folgendes Reaktionsschema wiedergegeben werden:

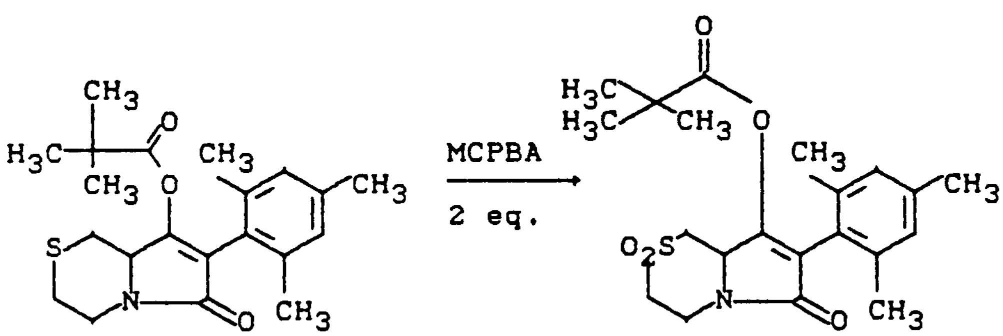

Die bei dem obigen Verfahren (A) als Ausgangsstoffe benötigten Verbindungen der Formel (II),

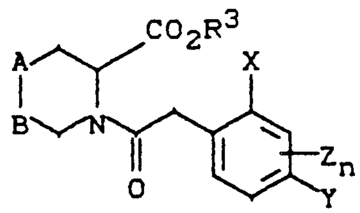

(II)

in welcher
A, B, X, Y, Z, n und $R^3$ die oben angegebene Bedeutung haben, sind nicht bekannt, lassen sich aber nach im Prinzip bekannten Methoden in einfacher Weise herstellen. So erhält man z.B. Acyl-aminosäureester der Formel (II), wenn man

a) Aminosäureester der Formel (VI),

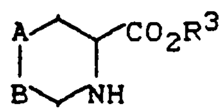

(VI)

in welcher

A und B die oben angegebene Bedeutung haben und
$R^3$ für Alkyl steht,

mit Phenylessigsäurehalogeniden der Formel (VII),

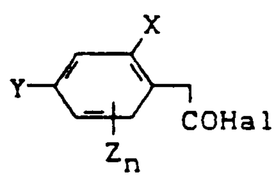

(VII)

in welcher

X, Y, Z und n die oben angegebene Bedeutung haben und
Hal für Chlor oder Brom steht,

acyliert (Allgemeine Methodik beschrieben in: Chem. Reviews 52 237-416 (1953));
oder wenn man

b) Acylaminosäuren der Formel (IIa),

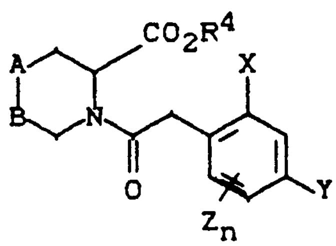

(IIa)

in welcher

A, B, X, Y, Z und n die oben angegebene Bedeutung haben
und
$R^4$ für Wasserstoff steht,

verestert (Allgemeine Methodik beschrieben in: Chem. Ind. (London) 1568 (1968)).

Verbindungen der Formel (IIa) sind beispielsweise aus den Phenylessigsäurehalogeniden der Formel (VII) und Aminosäuren der Formel (VIa),

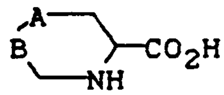

(VIa)

in welcher

A und B die oben angegebene Bedeutung haben,

nach Schotten-Baumann (Organikum 9. Auflage 44) (1970) VEB Deutscher Verlag der Wissenschaften, Berlin) erhältlich.

Verbindungen der Formel (VI) und (VIa) sind bekannte Verbindungen und lassen sich in einfacher Weise darstellen.

Beispielhaft sind folgende Verbindungen der Formel (II) genannt:

1) N-(2,4-Dichlorphenylacetyl)-1,4-thiomorpholin-3-carbonsäureethylester
2) N-(2,6-Dichlorphenylacetyl)-1,4-thiomorpholin-3-carbonsäureethylester
3) N-(2,4,6-Trichlorphenylacetyl)-1,4-thiomorpholin-3-carbonsäureethylester
4) N-(2,4-Dimethylphenylacetyl)-1,4-thiomorpholin-3-carbonsäureethylester
5) N-(2,6-Dimethylphenylacetyl)-1,4-thiomorpholin-3-carbonsäureester
6) N-(2,4,6-Trimethylphenylacetyl)-1,4-thiomorpholin-3-carbonsäureester
7) N-(2-Chlor-6-fluorphenylacetyl)-1,4-thiomorpholin-3-carbonsäureethylester
8) N-(2,6-Dichlor-4-trifluormethylphenylacetyl)-1,4-thiomorpholin-3-carbonsäureethylester
9) N-(2,4-Dichlorphenylacetyl)-1,3-thiomorpholin-4-carbonsäureethylester
10) N-(2,6-Dichlorphenylacetyl)-1,3-thiomorpholin-4-carbonsäureethylester
11) N-(2,4,6-Trichlorphenylacetyl)-1,3-thiomorpholin-4-carbonsäureethylester
12) N-(2,4-Dimethylphenylacetyl)-1,3-thiomorpholin-4-carbonsäureethylester
13) N-(2,6-Dimethylphenylacetyl)-1,3-thiomorpholin-4-carbonsäureethylester
14) N-(2,4,6-Trimethylphenylacetyl)-1,3-thiomorpholin-4-carbonsäureethylester
15) N-(2-Chlor-6-fluorphenylacetyl)-1,3-thiomorpholin-4-carbonsäureethylester
16) N-(2,6-Dichlor-4-trifluormethylphenylacetyl)-1,3-thiomorpholin-4-carbonsäureethylester
17) N-(2,4-Dichlorphenylacetyl)-thiazolidin-4-carbonsäureethylester
18) N-(2,6-Dichlorphenylacetyl)-thiazolidin-4-carbonsäureethylester
19) N-(2,4,6-Trichlorphenylacetyl)-thiazolidin-4-carbonsäureethylester
20) N-(2,4-Dimethylphenylacetyl)-thiazolidin-4-carbonsäureethylester
21) N-(2,6-Dimethylphenylacetyl)-thiazolidin-4-carbonsäureethylester
22) N-(2,4,6-Trimethylphenylacetyl)-thiazolidin-4-carbonsäureethylester
23) N-(2-Chlor-6-fluorphenylacetyl)-thiazolidin-4-carbonsäureethylester
24) N-(2,6-Dichlor-4-trifluormethylphenylacetyl)-4-thiazolidin-carbonsäureethylester.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0 °C und 250 °C, vorzugsweise zwischen 50 °C und 150 °C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formeln (II) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (A) ist dadurch gekennzeichnet, daß Verbindungen der Formel (II) in welcher X, Y, Z, m, n und $R^3$ die obenangegebene Bedeutung haben in Gegenwart von Basen einer intramolekularen Kondensation unterwirft.

Als Verdünnungsmittel können bei dem erfindungsgemäßen Verfahren (A) alle üblichen inerten organischen Solventien eingesetzt werden. Vorzugsweise verwendbar sind Kohlenwasserstoffe, wie Cyclohexan, Toluol und Xylol, ferner Ether, wie Dibutylether, Tetrahydrofuran, Dioxan, Glylkoldimethylether und Diglykoldimethylether, außerdem polare Lösungsmittel, wie Dimethylsulfoxid, Sulfolan, Dimethylformamid und N-Methyl-pyrrolidon.

Als Deprotonierungsmittel können bei der Durchführung des erfindungsgemäßen Verfahrens (A) alle üblichen Protonenakzeptoren eingesetzt werden. Vorzugsweise verwendbar sind Alkalimetall- und Erdalkali-

metall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464oder TDA 1eingesetzt werden können. Ferner sind Alkalimetall- und Erdalkalimetallamide und -hydride, wie Natriumamid, Natriumhydrid und Calciumhydrid und außerdem auch Alkalimetall-alkoholate wie Natrium-methylat, Natriummethylat und Kalium-tert.-butylat einsetzbar.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Verwendet man die entsprechenden Carbonsäurehalogenide, so kommen als Säurebindemittel bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Die Reaktionstemperaturen können auch bei dem erfindungsgemäßen Verfahren (Bα) auch bei der Verwendung von Carbonsäurehalogeniden innerhalb eines größeren Bereiches variiert werden. Im allgemeinen arbeitet man bei Temperaturen zwischen -20 °C und +150 °C, vorzugsweise zwischen 0 °C und 100 °C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäurehydriden der Formel (IV) umsetzt.

Verwendet man bei dem erfindungsgemäßen Verfahren (Bβ) als Reaktionskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäurehydrid gleichzeitig als Verdünnungsmittel fungieren.

Die Reaktionstemperaturen können auch bei dem erfindungsgemäßen Verfahren (Bβ) auch bei der Verwendung von Carbonsäureanhydriden innerhalb eines größeren Bereiches variiert werden. Im allgemeinen arbeitet man bei Temperaturen zwischen -20 °C und +150 °C, vorzugsweise zwischen 0 °C und 100 °C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäurenhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern der Formel (V) umsetzt.

Verwendet man die entsprechenden Chlorameisensäureester so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBC, DBA, Hünig-Base und N,N-Dimethyl-anilin, ferner Erdalkalimetalloxide, wie Magnesium- und Calcium-oxid, außerdem Alkali- und

Adogen 464 = Methyltrialkyl($C_8$-$C_{10}$)ammoniumchlorid

TDA 1 = Tris-(methoxyethoxylethyl)-amin

Erdalkali-metall-carbonate, wie Natriumcarbonat, Kaliumcarbonat und Calciumcarbonat.

Als Verdünnungsmittel können bei dem erfindungsgemäßen Verfahren (C) bei Verwendung der Chlorameisensäureester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe wie Methylenchlorid, Chloroform Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester wie Ethylacetat und auch stark polare Solventien, wie Dimethylsulfoxid und Sulfolan.

Bei Verwendung der Chlorameisensäureester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20 °C und +100 °C, vorzugsweise zwischen 0 °C und 50 °C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende (Chlorameisensäureester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Das Verfahren (D) ist dadurch gekennzeichnet, daß man Verbindungen der Formel (Ia) mit Acetalhydroxiden (VIII) oder Aminen(IX) umsetzt.

Als Verdünnungsmittel können bei dem erfindungsgemäßen Verfahen vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (D) wird im allgemeinen unter Normaldruck durchgeführt. Die Reaktionstemperaturen liegen im allgemeinen zwischen -20 °C und 100 °C, vorzugsweise zwischen 0 °C und 50 °C.

Bei der Durchführung des erfindungsgemäßen Verfahrens (D) werden die Ausgangsstoffe der Formel (Ia) bzw. (IX) im allgemeinen in angenähert äquimolaren Mengen verwendet. Es ist jedoch auch möglich, die eine oder andere Komponente in einem größeren Überschuß (bis zu 2 Mol) einzusetzen. Im allgmeinen geht man so vor, daß man das Reaktionsgemisch durch Abziehen des Verdünnungsmittels einengt.

Bei der Durchführung des erfindungsgemäßen Verfahrens (E) werden die Ausgangsstoffe der Formel (Ia) bis (Ic) und das entsprechende Oxidationsmittel in angenähert äquimolaren Mengen eingesetzt. Die Aufarbeitung erfolgt nach den üblichen Methoden.

Als Oxidationsmittel kommen für das erfindungsgemäße Verfahren (E) alle schwefeloxidierenden Reagenzien in Frage, z.B. Halogen wie Chlor und Brom und deren wäßrige Lösungen, Alkaliperoxide wie Natriumperoxid und Kaliumperoxid, Salze von Halogensauerstoffsäuren wie Kaliumchlorat, Kaliumbromat, Natriumperjodat und Natriumperborat, weiterhin anorganische Persalze wie Kaliumpermanganat, Kaliumperoxodisulfat und Kaliumperoxomonosulfat, aber auch $H_2O_2$ in Gegenwart von Übergangsmetallsalzen wie Natriumwolframat und Ammoniummolybdat. Weiterhin verwendbar sind organische Peroxide wie tert.-Butylhydroperoxid aber auch organische Persäuren wie Peressigsäure, Perpropionsäure und m-Chlorperbenzoesäure (MCPBA).

Als Verdünnungsmittel können bei dem erfindungsgemäßen Verfahren (E) alle gegenüber diesen Verbindungen inerten Solventien eingesetzt werden. Vorzugsweise verwendbar sind Kohlenwasserstoffe wie Benzin, Benzol, Toluol, Xylol und Tetralin ferner Halogenkohlenwasserstoffe wie Methylenchlorid, Chloroform, Chlorbenzol und Dichlorbenzol, außerdem organische Säuren wie Essigsäure und Propionsäure und Wasser.

Arbeitet man in Gegenwart eines Verdünnungsmittels, so können die Reaktionstemperaturen bei der Durchführung des erfindungsgemäßen Verfahrens (E) innerhalb eines größeren Bereiches variiert werden. Im allgemeinen liegen die Reaktionstemperaturen zwischen -30 °C und +150 °C, vorzugsweise zwischen 0 °C und 100 °C.

Das erfindungsgemäße Verfahren (E) wird im allgemeinen unter Normaldruck durchgeführt.

Bei der Durchführung des erfindungsgemäßen Verfahrens (F) werden die Ausgangsstoffe der Formel (Ia) bis (Ic) und das entsprechende Oxidationsmittel in angenähert doppelt äquimolaren Mengen eingesetzt. Es ist jedoch auch möglich, das Oxidationsmittel in einem größeren Überschuß (bis zu 2 Mol) einzusetzen. Die Aufarbeitung erfolgt nach den üblichen Methoden.

Als Oxidationsmittel kommen für das erfindungsgemäße Verfahren (F) alle schwefeloxidierenden Reagenzien in Frage, z.B. Halogen wie Chlor und Brom und deren wäßrige Lösungen, Alkaliperoxide wie

Natriumperoxid und Kaliumperoxid, Salze von Halogensauerstoffsäuren wie Kaliumchlorat, Kaliumbromat, Natriumperjodat und Natriumperborat, weiterhin anorganische Persalze wie Kaliumpermanganat, Kaliumperoxodisulfat und Kaliumperoxomonosulfat, aber auch $H_2O_2$ in Gegenwart von Übergangsmetallsalzen wie Natriumwolframat und Ammoniummolybdat. Weiterhin verwendbar sind organische Peroxide wie tert.-Butylhydroperoxid aber auch organische Persäuren wie Peressigsäure, Perpropionsäure und m-Chlorperbenzoesäure (MCPBA).

Als Verdünnungsmittel können bei dem erfindungsgemäßen Verfahren (F) alle gegenüber diesen Verbindungen inerten Solventien eingesetzt werden. Vorzugsweise verwendbar sind Kohlenwasserstoffe wie Benzin, Benzol, Toluol, Xylol und Tetralin, ferner Halogenkohlenwasserstoffe wie Methylenchlorid, Chloroform, Chlorbenzol und Dichlorbenzol, außerdem organische Säuren wie Essigsäure und Propionsäure und Wasser.

Arbeitet man in Gegenwart eines Verdünnungsmittels, so können die Reaktionstemperaturen bei der Durchführung des erfindungsgemäßen Verfahrens (F) innerhalb eines größeren Bereiches variiert werden. Im allgemeinen liegen die Reaktionstemperaturen zwischen -30 °C und +150 °C, vorzugsweise zwischen 0 °C und 100 °C.

Das erfindungsgemäße Verfahren (F) wird im allgemeinen unter Normaldruck durchgeführt.

Beispiel 1

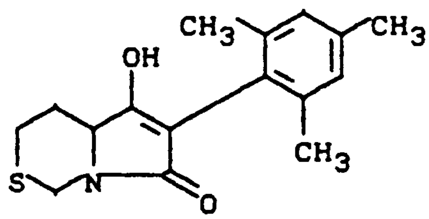

5,17 g (0,17 Mol) Natriumhydrid werden in 90 ml abs. Toluol vorgelegt. Unter Rückfluß werden 43 g (ca. 0,128 Mol) N-(2,4,6-Trimethylphenylacetyl)-1,3-thiomorpholin-4-carbonsäureethylester, gelöst in 130 ml absolutem (abs.) Toluol, zugetropft. Nach 3 h Rückflussieren tropft man unter Eisbadkühlung solange EtOH zu, bis die $H_2$-Entwicklung beendet ist. Es wird im Vakuum einrotiert, der Rückstand in Wasser aufgenommen, bei 0 bis 10 °C mit konz. HCl angesäuert, das Rohprodukt abgesaugt, und bei 70 °C im Vakuum über $P_2O_5$ getrocknet. Die Reinigung erfolgt durch Auskochen mit $CHCl_3$/MTB-Ether/n-Hexan.
Ausbeute: 32,06 g (86,5 % der Theorie) Fp. 294 °C.

Beispiel 2

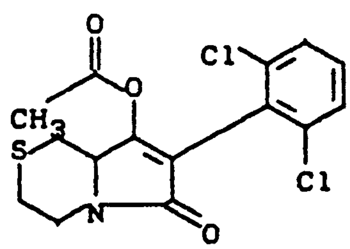

6,32 g (20 mmol) 3-(2,6-Dichlorphenyl)-1,5-ethylthiomethylpyrrolidin-2,4-dion werden in 50 ml tert.-Butylmethyl-Ether (MTB-Ether) suspendiert, mit 1,63 ml (20 mmol) abs. Pyridin und 3,4 ml (20 mmol) Ethyldiisopropylamin versetzt. Bei 0 bis 10 °C tropft man 1,5 ml (20 mmol) Acetylchlorid, gelöst in 5 ml MTB-Ether, zu, rührt 30 Min. bei Raumtemperatur nach, filtriert ab, engt im Vakuum ein, reinigt den Rückstand durch Säulenchromatographie an Kieselgel mit Cyclohexan/Essigester 1:1 und kristallisiert das Produkt aus n-Hexan aus.
Ausbeute: 3,7 g (51,6 % der Theorie) Fp. 112 °C.

Beispiel 3

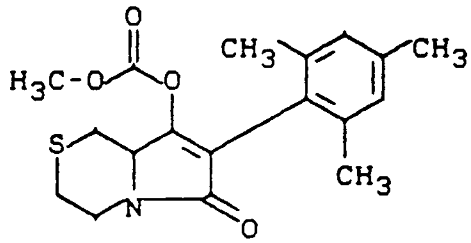

5,78 g (20 mmol) 3-(2,4,6-Trimethyl)-1,5-ethylthiomethylpyrrolidin-2,4-dion werden in 50 ml MTB-Ether suspendiert, mit 1,63 ml (20 mmol) abs. Pyridin und 3,4 ml (20 mmol) Ethyldiisopropylamin versetzt. Bei 0 bis 10 ° C tropft man 1,55 ml (20 mmol) Chlorameisensäuremethylester, gelöst in 5 ml MTB-Ether, zu, rührt 30 Min. bei Raumtemperatur nach, filtriert ab, engt im Vakuum ein und reinigt den Rückstand durch Säulenchromatographie an Kieselgel mit Cyclohexan/Essigester 1:1. Man erhält 5,26 g (75,7 % der Theorie) eines farblosen Öls mit einem gaschromatographisch bestimmten Gehalt von 95 %.
$^1$H-NMR (200 MHz, $CDCl_3$): $\delta$ = 2.13, 2.14 (2s, 6H, Ar-2-$CH_3$, Ar-6-$CH_3$), 2.28 (s, 3H, Ar-4-$CH_3$), 2.43-2.8 (m, 3H, <u>$CH_2$</u>-S-<u>$CH_2$</u>), 2.97-3.25 (m, 2H), 4.57 (ABq, 1H), 4.7

("dt", 1H, N-CH-C=)

6.9 (s, 2H, Ar-3H, 6-H).

Beispiel 4

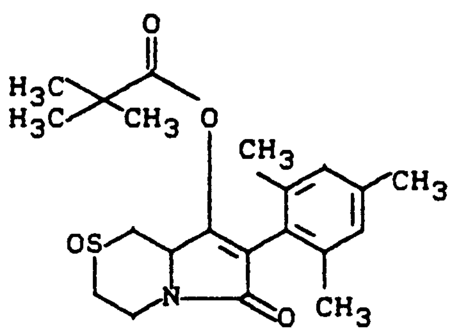

7,47 g (20 mmol) 4-Pivaloyloxy-3-(2,4,6-trimethylphenyl)-1,5-ethylmercaptomethyl-3-pyrrolin-2-on werden in 60 ml Chloroform gelöst und bei 0 bis 5 ° C wird eine Lösung von 4,2 g (20 mmol) m-Chlorperbenzoesäure in 50 ml Chloroform zugetropft. Nach 1 h Rühren bei 0 ° C wäscht man die Lösung 2-mal mit $NaHCO_3$-Lösung, trocknet, engt im Vakuum ein und kristallisiert aus Methylenchlorid/n-Hexan um.
Ausbeute: 2,95 g (38 % der Theorie) Fp. 217 ° C.

Beispiel 5

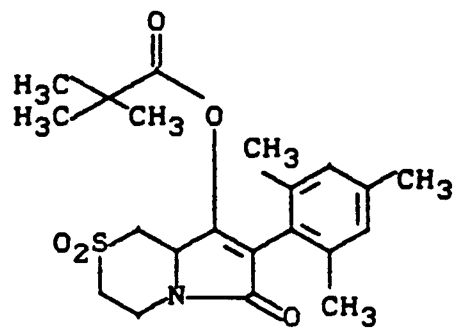

7,47 g (20 mmol) 4-Pivaloyloxy-3-(2,4,6-trimethylphenyl)-1,5-ethylmercaptomethyl-3-pyrrolin-2-on werden in 60 ml Chloroform gelöst und bei 0 bis 5 ° C wird eine Lösung von 9,24 g (44 mmol) m-Chlorperbenzoesäure in 100 ml Chloroform zugetropft. Nach 1 h Rühren bei 0 ° C wäscht man die Lösung 2-mal mit $NaHCO_3$-Lösung, trocknet, engt im Vakuum ein und kristallisiert aus Methylenchlorid/n-Hexan um.
Ausbeute: 5,7 g (70,3 % der Theorie) Fp. 235 ° C.

In entsprechender Weise zu den Herstellungsbeispielen und gemäß den allgemeinen Angaben zur Herstellung erhält man die in den nachfolgenden Tabellen 1 bis 3 formelmäßig aufgeführten 3-Aryl-pyrrolidin-2,4-dion-Derivate der Formel (Ia) bis (Ie).

Tabelle 1

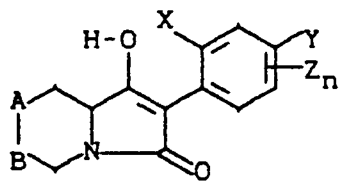

(Ia)

| Nr. | A - B | X | Y | $Z_n$ | Fp.°C |
|---|---|---|---|---|---|
| 6 | -S- | Cl | Cl | H | 220 |
| 7 | -SO- | Cl | Cl | H | |
| 8 | $-SO_2-$ | Cl | Cl | H | |
| 9 | $-S-CH_2-$ | Cl | Cl | H | 225 |
| 10 | $-SO-CH_2-$ | Cl | Cl | H | |
| 11 | $-SO_2-CH_2-$ | Cl | Cl | H | |
| 12 | $-CH_2-S-$ | Cl | Cl | H | 205 |
| 13 | $-CH_2-SO-$ | Cl | Cl | H | |
| 14 | $-CH_2-SO_2-$ | Cl | Cl | H | |
| 15 | -S- | Cl | H | 6-Cl | |
| 16 | -SO- | Cl | H | 6-Cl | |
| 17 | $-SO_2-$ | Cl | H | 6-Cl | |
| 18 | $-S-CH_2-$ | Cl | H | 6-Cl | >230 |
| 19 | $-SO-CH_2-$ | Cl | H | 6-Cl | |
| 20 | $-SO_2-CH_2-$ | Cl | H | 6-Cl | |
| 21 | $-CH_2-S-$ | Cl | H | 6-Cl | |
| 22 | $-CH_2-SO-$ | Cl | H | 6-Cl | |
| 23 | $-CH_2-SO_2-$ | Cl | H | 6-Cl | |
| 24 | -S- | $CH_3$ | $CH_3$ | $6-CH_3$ | 221 |
| 25 | -SO- | $CH_3$ | $CH_3$ | $6-CH_3$ | |
| 26 | $-SO_2-$ | $CH_3$ | $CH_3$ | $6-CH_3$ | |
| 27 | $-S-CH_2-$ | $CH_3$ | $CH_3$ | $6-CH_3$ | 231 |
| 28 | $-SO-CH_2-$ | $CH_3$ | $CH_3$ | $6-CH_3$ | |
| 29 | $-SO_2-CH_2-$ | $CH_3$ | $CH_3$ | $6-CH_3$ | |
| 30 | $-CH_2-SO-$ | $CH_3$ | $CH_3$ | $6-CH_3$ | |
| 31 | $-CH_2-SO_2-$ | $CH_3$ | $CH_3$ | $6-CH_3$ | |

Tabelle 2

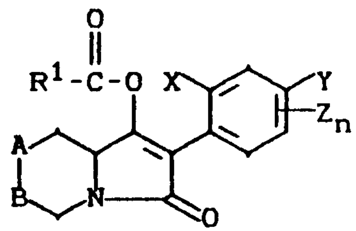

(Ib)

| Nr. | A - B | X | Y | $Z_n$ | $R^1$ | Fp. °C |
|---|---|---|---|---|---|---|
| 32 | -S- | Cl | Cl | H | $CH_3$ | |
| 33 | -S- | Cl | Cl | H | $(CH_3)_3C-$ | |
| 34 | -S- | Cl | Cl | H | $(CH_3)_2CH-C(CH_3)_2-$ | Öl |
| 35 | -SO- | Cl | Cl | H | $(CH_3)_3C-$ | |
| 36 | $-SO_2-$ | Cl | Cl | H | $(CH_3)_3C-$ | |
| 37 | -S- | Cl | H | 6-Cl | $CH_3-$ | |
| 38 | -S- | Cl | H | 6-Cl | $(CH_3)_3C-$ | |
| 39 | -S- | $CH_3$ | $CH_3$ | 6-$CH_3$ | $CH_3-$ | |
| 40 | -S- | $CH_3$ | $CH_3$ | 6-$CH_3$ | $(CH_3)_2CH-$ | |
| 41 | -S- | $CH_3$ | $CH_3$ | 6-$CH_3$ | $(CH_3)_3C-$ | |
| 42 | -S- | $CH_3$ | $CH_3$ | 6-$CH_3$ | $(CH_3)_2CH-C(CH_3)_2-$ | |

Tabelle 2 (Fortsetzung)

| Nr. | A - B | X | Y | $Z_n$ | $R^1$ | Fp.°C |
|---|---|---|---|---|---|---|
| 43 | $-SO-$ | $CH_3$ | $CH_3$ | $6-CH_3$ | $CH_3-$ | |
| 44 | $-SO-$ | $CH_3$ | $CH_3$ | $6-CH_3$ | $(CH_3)_3C-$ | |
| 45 | $-SO_2-$ | $CH_3$ | $CH_3$ | $6-CH_3$ | $CH_3-$ | |
| 46 | $-SO_2-$ | $CH_3$ | $CH_3$ | $6-CH_3$ | $(CH_3)_3C-$ | |
| 47 | $-S-CH_2-$ | Cl | Cl | H | $CH_3-$ | |
| 48 | $-S-CH_2-$ | Cl | Cl | H | $(CH_3)_3C-$ | |
| 49 | $-S-CH_2-$ | Cl | Cl | H | $(CH_3)_2CH-(CH_3)_2C-$ | Öl |
| 50 | $-SO-CH_2-$ | Cl | Cl | H | $(CH_3)_3C-$ | |
| 51 | $-SO_2-CH_2-$ | Cl | Cl | H | $(CH_3)_3C-$ | |
| 52 | $-S-CH_2-$ | Cl | H | 6-Cl | $(CH_3)_2CH-$ | |
| 53 | $-S-CH_2-$ | Cl | H | 6-Cl | $(CH_3)_3C-$ | Öl |
| 54 | $-S-CH_2$ | Cl | H | 6-Cl | $(CH_3)_2CH-(CH_3)_2C-$ | |
| 55 | $-SO-CH_2$ | Cl | H | 6-Cl | $(CH_3)_3C-$ | |
| 56 | $-SO_2-CH_2-$ | Cl | H | 6-Cl | $(CH_3)_3C-$ | |
| 57 | $-S-CH_2-$ | $CH_3$ | $CH_3$ | $6-CH_3$ | $CH_3-$ | Glas |
| 58 | $-S-CH_2-$ | $CH_3$ | $CH_3$ | $6-CH_3$ | $(CH_3)_2CH-$ | Öl |
| 59 | $-S-CH_2-$ | $CH_3$ | $CH_3$ | $6-CH_3$ | $(CH_3)_3C-$ | 110 |

T a b e l l e 2 (Fortsetzung)

| Nr. | A - B | X | Y | $Z_n$ | $R^1$ | Fp.°C |
|---|---|---|---|---|---|---|
| 60 | $-S-CH_2-$ | $CH_3$ | $CH_3$ | $6-CH_3$ | $(CH_3)_2CH-(CH_3)_2C-$ | 74 |
| 61 | $-S-CH_2-$ | $CH_3$ | $CH_3$ | $6-CH_3$ | $(CH_3)_3C-CH_2-$ | Öl |
| 62 | $-S-CH_2-$ | $CH_3$ | $CH_3$ | $6-CH_3$ | $Cl-CH_2-C(CH_3)_2-$ | 118 |
| 63 | $-S-CH_2-$ | $CH_3$ | $CH_3$ | $6-CH_3$ | $CH_3O-CH_2-(CH_3)_2C-$ | Öl |
| 64 | $-S-CH_2-$ | $CH_3$ | $CH_3$ | $6-CH_3$ | $(CH_3)_2C=CH-$ | Öl |
| 65 | $-S-CH_2-$ | $CH_3$ | $CH_3$ | $6-CH_3$ | 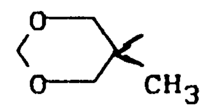 | 136 |
| 66 | $-S-CH_2-$ | $CH_3$ | $CH_3$ | $6-CH_3$ | 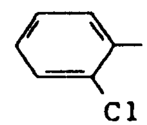 | |
| 67 | $-S-CH_2-$ | $CH_3$ | $CH_3$ | $6-CH_3$ | 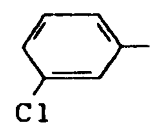 | 130 |
| 68 | $-S-CH_2-$ | $CH_3$ | $CH_3$ | $6-CH_3$ | 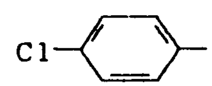 | 145 |
| 69 | $-SO-CH_2-$ | $CH_3$ | $CH_3$ | $6-CH_3$ | $CH_3-$ | |

T a b e l l e 2 (Fortsetzung)

| Nr. | A - B | X | Y | $Z_n$ | $R^1$ | Fp.°C |
|---|---|---|---|---|---|---|
| 70 | $-SO-CH_2-$ | $CH_3$ | $CH_3$ | $6-CH_3$ | $(CH_3)_3C-CH_2-$ | |
| 71 | $-SO_2-CH_2-$ | $CH_3$ | $CH_3$ | $6-CH_3$ | $CH_3-$ | |
| 72 | $-SO_2-CH_2-$ | $CH_3$ | $CH_3$ | $6-CH_3$ | $(CH_3)_3C-CH_2-$ | |
| 73 | $-CH_2-S-$ | $CH_3$ | $CH_3$ | $6-CH_3$ | $CH_3-$ | 164 |
| 74 | $-CH_2-S-$ | $CH_3$ | $CH_3$ | $6-CH_3$ | $(CH_3)_2CH-$ | 84 |
| 75 | $-CH_2-S-$ | $CH_3$ | $CH_3$ | $6-CH_3$ | $(CH_3)_3C-$ | 123 |
| 76 | $-CH_2-S-$ | $CH_3$ | $CH_3$ | $6-CH_3$ | $(CH_3)_2CH-(CH_3)_2C-$ | 113 |
| 77 | $-CH_2-SO-$ | $CH_3$ | $CH_3$ | $6-CH_3$ | $CH_3-$ | |
| 78 | $-CH_2-SO-$ | $CH_3$ | $CH_3$ | $6-CH_3$ | $(CH_3)_3C-$ | |
| 79 | $-CH_2-S-$ | Cl | Cl | H | $CH_3-$ | 139 |
| 80 | $-CH_2-S-$ | Cl | Cl | H | $(CH_3)_2CH-(CH_3)_2C-$ | 133 |

T a b e l l e 3

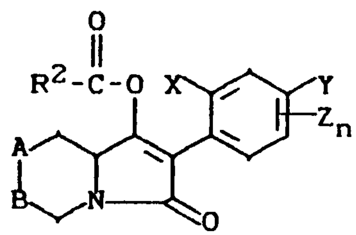

(Ic)

| Nr. | A - B | X | Y | $Z_n$ | $R^2$ | Fp.°C |
|---|---|---|---|---|---|---|
| 81 | $-S-$ | Cl | Cl | H | $CH_3CH_2-$ | |
| 82 | $-S-$ | Cl | Cl | H | $(CH_3)_2CH-$ | |
| 83 | $-SO-$ | Cl | Cl | H | $CH_3CH_2-$ | |
| 84 | $-SO_2-$ | Cl | Cl | H | $CH_3CH_2-$ | |
| 85 | $-S-CH_2-$ | Cl | Cl | H | $CH_3CH_2-$ | |
| 86 | $-S-CH_2-$ | Cl | Cl | H | $(CH_3)_2CH-$ | |
| 87 | $-SO-CH_2-$ | Cl | Cl | H | $CH_3CH_2-$ | |
| 88 | $-SO_2-CH_2-$ | Cl | Cl | H | $CH_3CH_2-$ | |
| 89 | $-CH_2-S-$ | Cl | Cl | H | $CH_3CH_2-$ | |
| 90 | $-CH_2-S-$ | Cl | Cl | H | $(CH_3)_2CH-$ | |
| 91 | $-CH_2-SO-$ | Cl | Cl | H | $CH_3CH_2-$ | |

T a b e l l e 3 (Fortsetzung)

| Nr. | A - B | X | Y | $Z_n$ | $R^2$ | Fp. °C |
|---|---|---|---|---|---|---|
| 92 | $-CH_2-SO_2-$ | Cl | Cl | H | $CH_3CH_2-$ | |
| 93 | -S- | Cl | H | 6-Cl | $CH_3CH_2-$ | |
| 94 | -S- | Cl | H | 6-Cl | $(CH_3)_2CH-$ | |
| 95 | -SO- | Cl | H | 6-Cl | $CH_3-CH_2-$ | |
| 96 | $-SO_2-$ | Cl | H | 6-Cl | $CH_3CH_2-$ | |
| 97 | $-S-CH_2-$ | Cl | H | 6-Cl | $CH_3-$ | |
| 98 | $-S-CH_2-$ | Cl | H | 6-Cl | $CH_3CH_2-$ | 95 |
| 99 | $-S-CH_2-$ | Cl | H | 6-Cl | $(CH_3)_2CH-$ | |
| 100 | $-S-CH_2-$ | Cl | H | 6-Cl | $(CH_3)_2CH-CH_2-$ | |
| 101 | $-S-CH_2-$ | Cl | H | 6-Cl | $CH_3\backslash$<br>$CH-$<br>$C_2H_5/$ | |
| 102 | $-S-CH_2-$ | Cl | H | 6-Cl | $(CH_3)_3C-CH_2-$ | |
| 103 | $-SO-CH_2-$ | Cl | H | 6-Cl | $C_2H_5-$ | |
| 104 | $-SO-CH_2-$ | Cl | H | 6-Cl | $(CH_3)_2CH-$ | |
| 105 | $-SO_2-CH_2-$ | Cl | H | 6-Cl | $C_2H_5-$ | |

T a b e l l e 3 (Fortsetzung)

| Nr. | A - B | X | Y | $Z_n$ | $R^2$ | Fp.°C |
|---|---|---|---|---|---|---|
| 106 | $-SO_2-CH_2-$ | Cl | H | 6-Cl | $(CH_3)_2CH-$ | |
| 107 | $-CH_2-S-$ | Cl | H | 6-Cl | $C_2H_5-$ | |
| 108 | $-CH_2-S-$ | Cl | H | 6-Cl | $(CH_3)_2CH-$ | |
| 109 | $-CH_2-SO-$ | Cl | H | 6-Cl | $C_2H_5-$ | |
| 110 | $-CH_2-SO_2-$ | Cl | H | 6-Cl | $C_2H_5-$ | |
| 111 | -S- | $CH_3$ | $CH_3$ | $6-CH_3$ | $C_2H_5-$ | |
| 112 | -S- | $CH_3$ | $CH_3$ | $6-CH_3$ | $C_2H_5-$ | |
| 113 | -SO- | $CH_3$ | $CH_3$ | $6-CH_3$ | $C_2H_5-$ | |
| 114 | $-SO_2-$ | $CH_3$ | $CH_3$ | $6-CH_3$ | $C_2H_5-$ | |
| 115 | $-S-CH_2-$ | $CH_3$ | $CH_3$ | $6-CH_3$ | $(CH_3)_3C-$ | |
| 116 | $-S-CH_2-$ | $CH_3$ | $CH_3$ | $6-CH_3$ | $(CH_3)_2CH-CH_2-$ | Öl |
| 117 | $-S-CH_2-$ | $CH_3$ | $CH_3$ | $6-CH_3$ | $CH_3$<br>CH-<br>$C_2H_5$ | Öl |
| 118 | $-S-CH_2-$ | $CH_3$ | $CH_3$ | $6-CH_3$ | $(CH_3)_3C-CH_2-$ | 95 |
| 119 | $-S-CH_2-$ | $CH_3$ | $CH_3$ | $6-CH_3$ | $C_2H_5-O-(CH_2)_2-$ | Öl |

<u>T a b e l l e 3</u> (Fortsetzung)

| Nr. | A - B | X | Y | $Z_n$ | $R^2$ | Fp.°C |
|---|---|---|---|---|---|---|
| 120 | $-S-CH_2-$ | $CH_3$ | $CH_3$ | $6-CH_3$ | 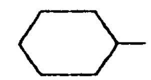 | Öl |
| 121 | $-S-CH_2-$ | $CH_3$ | $CH_3$ | $6-CH_3$ | 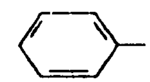 | |
| 122 | $-SO-CH_2-$ | $CH_3$ | $CH_3$ | $6-CH_3$ | $C_2H_5-$ | |
| 123 | $-SO-CH_2-$ | $CH_3$ | $CH_3$ | $6-CH_3$ | $(CH_3)_2CH-$ | |
| 124 | $-SO_2-CH_2-$ | $CH_3$ | $CH_3$ | $6-CH_3$ | $C_2H_5-$ | |
| 125 | $-SO_2-CH_2-$ | $CH_3$ | $CH_3$ | $6-CH_3$ | $(CH_3)_2CH-$ | |
| 126 | $-CH_2-S-$ | $CH_3$ | $CH_3$ | $6-CH_3$ | $C_2H_5-$ | |
| 127 | $-CH_2-S-$ | $CH_3$ | $CH_3$ | $6-CH_3$ | $(CH_3)_2CH-$ | |
| 128 | $-CH_2-SO-$ | $CH_3$ | $CH_3$ | $6-CH_3$ | $C_2H_5-$ | |
| 129 | $-CH_2-SO_2-$ | $CH_3$ | $CH_3$ | $6-CH_3$ | $C_2H_5-$ | |
| 130 | $-S-CH_2-$ | $CH_3$ | $CH_3$ | $6-CH_3$ | $C_2H_5-$ | Öl |
| 131 | $-S-CH_2$ | $CH_3$ | $CH_3$ | $6-CH_3$ | $(CH_3)_2CH-$ | Öl |

Zwischenprodukte

Beispiel I

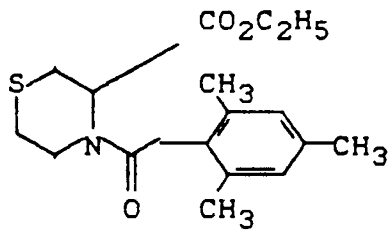

175,3 g (1 Mol) Thiomorpholin-3-carbonsäureethylester werden in 1000 ml abs. THF gelöst, mit 140 ml (1 Mol) Triethylamin versetzt und bei 0° bis 10°C 198,5 g (1 Mol) Mesitylenessigsäurechlorid zugetropft. Man rührt noch 1 h bei Raumtemperatur nach, gießt den Ansatz in 4,5 l Eiswasser und 500 ml 1N HCl und saugt ein flockiges, stark wasserhaltiges Produkt ab, das in Methylenchlorid aufgenommen, mit $MgSO_4$ getrocknet und einrotiert wird. Auf diese Weise erhält man 317,2 g (= 94,7 % der Theorie) N-(2,4,6-Trimethylphenyl-acetyl)-thiomorpholin-3-carbonsäureethylester als hellgelbes Öl.

$^1$H-NMR (200 MHz, $CDCl_3$): $\delta$ = 1.25 (t, 3H, $CH_2\underline{CH_3}$), 2.18 (s, 6H, Ar-2-$CH_3$, Ar-6-$CH_3$), 2.21 (s, 3H, Ar-4-$CH_3$), 4.2 (q, 2H, -$\underline{CH_2}$-$CH_3$), 5.68

("t", 1H, N-C$\underline{H}$-$CO_2C_2H_5$),

6.8 (s, 2H, Ar-3-$\underline{H}$, Ar-S-$\underline{H}$).

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis

chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp. Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Globodera ssp., Meloidogyne spp., Aphelenchoides spp., Longidorus spp., Xiphinema spp., Trichodorus spp..

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Charakteristisch für die erfindungsgemäßen Verbindungen ist, daß sie eine selektive Wirksamkeit gegen monokotyle Unkräuter in monokotylen und dikotylen Kulturen im Vor- und Nachlaufverfahren (Pre- und Postemergence) bei guter Kulturflanzenverträglichkeit aufweisen.

Die erfindungsgemäßen Wirkstoffe können z.B. bei den folgenden Pflanzen verwendet werden:

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Wirkstoffe neben einer hervorragenden Wirkung gegen Schadpflanzen gute Verträglichkeit gegenüber wichtigen Kulturpflanzen, wie z. B. Weizen, Baumwolle, Sojabohnen, Citrusfrüchten und Zuckerrüben, und können daher als selektive Unkrautbekämpfungsmittel eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden Herbiziden oder Fungiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Z.B. können die Wirkstoffe als solche, in Form ihrer Formulierungen oder den daraus durch weiteres Verdünnen bzw. durch Kombination mit emulgierbaren Ölen, oberflächenaktiven Substanzen und weiteren Additionen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können als Herbizide sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,1 und 5 kg pro ha.

Die erfindungsgemäß verwendbaren Wirkstoffe eignen sich auch zur Bekämpfung von Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Die Verwendung der erfindungsgemäßen Wirkstoffe geht aus den nachfolgenden Beispielen hervor.

Beispiel A

Tetranychus-Test (resistent)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebene Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik:

Beispiel B

Nephotettix-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebene Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen z.B. die folgenden Verbindungen der Herstellungsbeispiele überlegene Wirksamkeit gegenüber dem Stand der Technik:

Beispiel C

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Beispiel D

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

**Patentansprüche**

**1.** 3-Aryl-pyrrolidin-2,4-dion-Derivate der Formel (I)

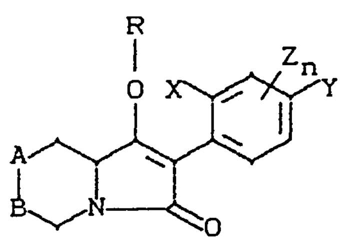

(I)

in welcher

A-B für $-S-CH_2-$, $-SO-CH_2-$, $-SO_2-CH_2-$, $-CH_2-S-$, $-CH_2-SO-$, $-CH_2-SO_2-$, -S-, -SO-, $-SO_2-$ steht,
X für $C_1$-$C_6$-Alkyl, Halogen, $C_1$-$C_6$-Alkoxy steht,
Y für Wasserstoff, $C_1$-$C_6$-Alkyl, Halogen, $C_1$-$C_6$-Alkoxy, $C_1$-$C_3$-Halogenalkyl steht,
Z für $C_1$-$C_6$-Alkyl, Halogen, $C_1$-$C_6$-Alkoxy steht,
n für eine Zahl von 0-3 steht,
R für Wasserstoff (Ia) oder für die Gruppen der Formel

$-CO-R^1$ (Ib), $-CO-O-R^2$ (Ic)

oder E (Id)

steht, in welchen

E für ein Metallkationäquivalent oder für ein Ammoniumion steht,
$R^1$ für gegebenenfalls durch Halogen substituiertes $C_1$-$C_{20}$-Alkyl, $C_2$-$C_{20}$-Alkenyl, $C_1$-$C_8$-Alkoxy-$C_1$-$C_8$-alkyl, $C_1$-$C_8$-Alkylthio-$C_1$-$C_8$-alkyl, $C_1$-$C_8$-Polyalkoxy-$C_2$-$C_8$-alkyl und Cycloalkyl mit 3-8 Ringatomen, das durch Sauerstoff und/oder Schwefel unterbrochen sein kann, steht,
für gegebenenfalls durch Halogen, Nitro, $C_1$-$C_6$-Alkyl, $C_1$-$C_6$-Alkoxy, $C_1$-$C_6$-Halogenalkyl, $C_1$-

$C_6$-Halogenalkoxy substituiertes Phenyl;
für gegebenenfalls durch Halogen, $C_1$-$C_6$-Alkyl, $C_1$-$C_6$-Alkoxy, $C_1$-$C_6$-Halogenalkyl, $C_1$-$C_6$-Halogenalkoxy substituiertes Phenyl-$C_1$-$C_6$-alkyl steht,
für gegebenenfalls durch Halogen- und $C_1$-$C_6$-Alkyl-substituiertes Pyridyl, Pyrimidyl, Thiazolyl und Pyrazolyl steht, für gegebenenfalls durch Halogen und $C_1$-$C_6$-Alkyl substituiertes Phenoxy-$C_1$-$C_6$-alkyl steht,
für gegebenenfalls durch Halogen, Amino und $C_1$-$C_6$-Alkyl substituiertes Pyridyloxy-$C_1$-$C_4$-alkyl, Pyrimidyloxy-$C_1$-$C_4$-alkyl und Thiazolyloxy-$C_1$-$C_4$-alkyl steht,

$R^2$ für gegebenenfalls durch Halogen substituiiertes $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Polyalkoxy-$C_2$-$C_8$-alkyl steht,
für gegebenenfalls durch Halogen, Nitro, $C_1$-$C_6$-Alkyl, $C_1$-$C_6$-Alkoxy, $C_1$-$C_6$-Halogenalkyl substituiertes Phenyl steht,

sowie die enantiomerenreinen Formen von Verbindungen der Formel (I).

**2.** 3-Aryl-pyrrolidin-2,4-2,4-dione der Formel (I) gemäß Anspruch 1, in welcher

A-B für -S-$CH_2$-, -SO-$CH_2$-, -$SO_2$-$CH_2$-, -$CH_2$-S-, -$CH_2$-SO-, -$CH_2$-$SO_2$-, -S-, -SO-, -$SO_2$- steht,
X für $C_1$-$C_4$-Alkyl, Halogen, $C_1$-$C_4$-Alkoxy steht,
Y für Wasserstoff, $C_1$-$C_6$-Alkyl, Halogen, $C_1$-$C_4$-Alkoxy, $C_1$-$C_2$-Halogenalkyl steht,
Z für $C_1$-$C_4$-Alkyl, Halogen, $C_1$-$C_4$-Alkoxy steht,
n für eine Zahl von 0-3 steht,
R für Wasserstoff (Ia) oder für die Gruppen der Formel

-CO-$R^1$ (Ib), -CO-O-$R^2$ (Ic)

oder E (Id)

steht, in welchen

E für ein Metallkationäquivalent oder für ein Ammoniumion steht,

$R^1$ für gegebenenfalls durch Halogen substituiertes $C_1$-$C_{16}$-Alkyl, $C_2$-$C_{16}$-Alkenyl, $C_1$-$C_6$-Alkoxy-$C_1$-$C_6$-alkyl, $C_1$-$C_6$-Alkylthio-$C_1$-$C_6$-alkyl, $C_1$-$C_6$-Polyalkoxy-$C_2$-$C_6$-alkyl und Cycloalkyl mit 3-7 Ringatomen, das durch 1-2 Sauerstoff- und/oder Schwefelatome unterbrochen sein kann steht,
für gegebenenfalls durch Halogen, Nitro, $C_1$-$C_4$-Alkyl, $C_1$-$C_4$-Alkoxy, $C_1$-$C_3$-Halogenalkyl, $C_1$-$C_3$-Halogenalkoxy substituiertes Phenyl steht,
für gegebenenfalls durch Halogen, $C_1$-$C_4$-Alkyl, $C_1$-$C_4$-Alkoxy, $C_1$-$C_3$-Halogenalkyl, $C_1$-$C_3$-Halogenalkoxy substituiertes Phenyl-$C_1$-$C_4$-alkyl steht,
für gegebenenfalls duch Halogen und $C_1$-$C_6$-Alkyl substituiertes Pyridyl, Pyrimidyl, Thiazolyl und Pyrazolyl steht,
gegebenenfalls für durch Halogen und $C_1$-$C_4$-Alkyl substituiertes Phenoxy-$C_1$-$C_5$-alkyl steht,
für gegebenenfalls durch Halogen, Amino und $C_1$-$C_4$-Alkyl substituiertes Pyridyloxy-$C_1$-$C_4$-alkyl, Pyrimidyloxy-$C_1$-$C_4$-alkyl und Thiazolyloxy-$C_1$-$C_4$-alkyl steht,

$R^2$ für gegebenenfalls durch Halogen substituiertes $C_1$-$C_{16}$-Alkyl, $C_2$-$C_{16}$-Alkenyl, $C_1$-$C_{16}$-Alkoxy-$C_2$-$C_6$-alkyl, $C_1$-$C_6$-Polyalkoxy-$C_2$-$C_6$-alkyl steht,
für gegebenenfalls durch Halogen, Nitro, $C_1$-$C_4$-Alkyl, $C_1$-$C_3$-Alkoxy, $C_1$-$C_3$-Halogenalkyl substituiertes Phenyl steht,

sowie die enantiomerenreinen Formen von Verbindungen der Formel (I).

**3.** 3-Aryl-pyrrolidin-2,4-dione der Formel (I) gemaß Anspruch 1, in welcher

A-B für -S-$CH_2$-, -SO-$CH_2$-, -$SO_2$-$CH_2$-, -$CH_2$-S-, -$CH_2$-SO-, -$CH_2SO_2$-, -S-, -SO-, -$SO_2$ steht
X für Methyl, Ethyl, Propyl, i-Propyl, Fluor, Chlor, Brom, Methoxy und Ethoxy steht,
Y für Wasserstoff, Methyl, Ethyl, Propyl, i-Propyl, Butyl, i-Butyl, tert.-Butyl, Chlor, Brom, Methoxy, Ethoxy und Trifluormethyl steht,
Z für Methyl, Ethyl, i-Propyl, Butyl, i-Butyl, tert.-Butyl, Chlor, Brom, Methoxy und Ethoxy steht,
n für eine Zahl von 0-3 steht,
R für Wasserstoff (Ia) oder für die Gruppen der Formel

-CO-$R^1$ (Ib), -CO-O-$R^2$ (Ic)

oder E (Id)

steht, in welcher

E für ein Metallkationäquivalent oder für ein Ammoniumion steht,

$R^1$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_1$-$C_6$-alkyl, $C_1$-$C_4$-Alkylthio-$C_1$-$C_6$-alkyl, $C_1$-$C_4$-Polyalkoxyl-$C_2$-$C_4$-alkyl und Cycloalkyl mit 3-6 Ringatomen, das durch 1-2 Sauerstoff- und/oder Schwefelatome unterbrochen sein kann steht,

für gegebenenfalls durch Fluor, Chlor, Brom, Methyl, Ethyl, Propyl, i-Propyl, Methoxy, Ethoxy, Trifluormethyl, Trifluormethoxy, Nitro substituiertes Phenyl steht,

für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-$C_1$-$C_3$-alkyl steht,

für gegebenenfalls durch Fluor, Chlor, Brom, Methyl, Ethyl substituiertes Pyridyl, Pyrimidyl, Thiazolyl und Pyrazolyl steht,

für gegebenenfalls durch Fluor, Chlor, Methyl, Ethyl substituiertes Phenoxy-$C_1$-$C_4$-alkyl steht,

für gegebenenfalls durch Fluor, Chlor, Amino, Methyl, Ethyl substituiertes Pyridyloxy-$C_1$-$C_4$-alkyl, Pyrimidyloxy-$C_1$-$C_4$-alkyl und Thiazolyloxy-$C_1$-$C_4$-alkyl steht,

$R^2$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Polyalkoxy-$C_2$-$C_6$-alkyl steht,

für gegebenenfalls durch Fluor, Chlor, Nitro, Methyl, Ethyl, Propyl, i-Propyl, Methoxy, Ethoxy, Trifluormethyl, substituiertes Phenyl steht,

sowie die enantiomerenreinen Formen von Verbindungen der Formel I.

**4.** Verfahren zur Herstellung von 3-Aryl-pyrrolidin-2,4-dion-Derivaten der Formel (I)

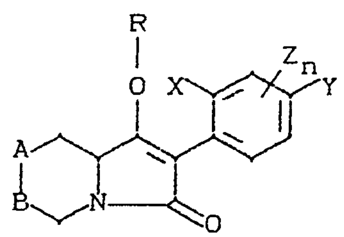

(I)

in welcher

A, B, R, X, Y, Z und n die in Anspruch 1 angegebene Bedeutung haben

sowie die enantiomerenreinen Formen von Verbindungen der Formel (I), dadurch gekennzeichnet, daß man

(A) zum Erhalt der Verbindungen der Formel (Ia)

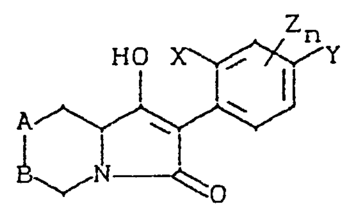

(Ia),

in welcher

A, B, X, Y, Z und n die oben angegebene Bedeutung haben,

N-Acylaminosäureester der Formel (II),

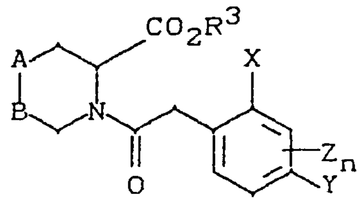

(II)

in welcher

A, B, X, Y, Z und n die oben angegebene Bedeutung haben

und

$R^3$ für Alkyl steht,

in Gegenwart eines Verdünnungsmittels und in Gegenwart einer Base intramolekular kondensiert,

(B) zum Erhalt von Verbindungen der Formel (Ib)

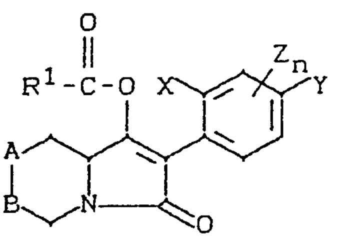

(Ib),

in welcher

A, B, X, Y, Z und n die oben angegebene Bedeutung haben,

Verbindungen der Formel (Ia)

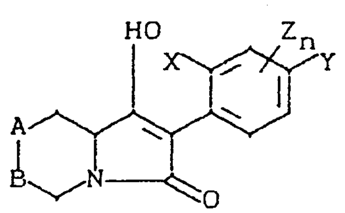

(Ia)

in welcher

A, B, X, Y, Z und n die oben angegebene Bedeutung haben,

α) mit Säurehalogeniden der allgemeinen Formel (III),

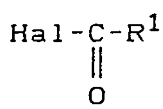

(III)

in welcher

$R^1$ die oben angegebene Bedeutung hat

und

Hal für Halogen, insbesondere Chlor und Brom steht,

gegebenenfalls in Gegenwart eines Verdünnungsmittels und gegebenenfalls in Gegenwart eines Saurebindemittels,

oder

β) mit Carbonsäureanhydriden der allgemeinen Formel (IV),

$R^1$-CO-O-CO-$R^1$ (IV)

in welcher
$R^1$ die oben angegebene Bedeutung hat,
gegebenenfalls in Gegenwart eines Verdünnungsmittels und gegebenenfalls in Gegenwart eines Saurebindemittels,
umsetzt,
(C) zum Erhalt von Verbindungen der Formel (Ic)

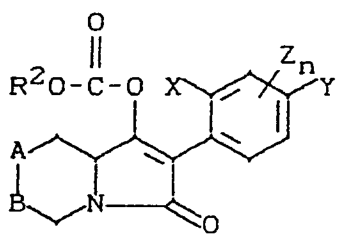

(Ic),

in welcher
A, B, X, Y, Z und n die oben angegebene Bedeutung haben
Verbindungen der Formel (Ia),

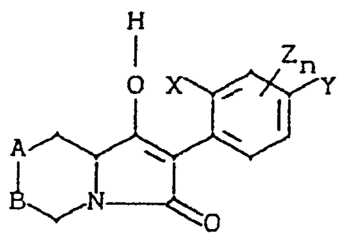

(Ia)

in welcher
A, B, X, Y, Z und n die oben angegebene Bedeutung haben
mit Chlorameisensäureester der allgemeinen Formel (V),

$R^2$-O-CO-Cl (V)

in welcher
$R^2$ die oben angegebene Bedeutung hat,
gegebenenfalls in Gegenwart eines Verdünnungsmittels und gegebenenfalls in Gegenwart eines Säurebindemittels umsetzt.
(D) zum Erhalt von Verbindungen der Formel (Id),

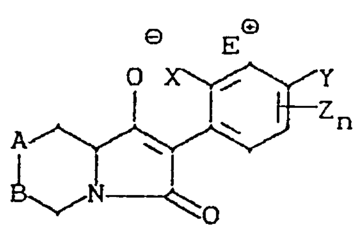

(Id)

in welcher
A, B, X, Y, Z, E und n die oben angegebene Bedeutung haben,
Verbindungen der Formel (Ia),

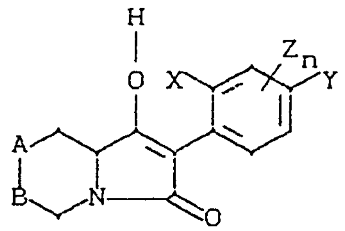

(Ia)

in welcher

A, B, X, Y, Z und n die oben angegebene Bedeutung haben,

mit Metallhydroxiden oder Aminen der allgemeinen Formeln (VIII) und (IX),

$Me_sOH_t$ (VIII)

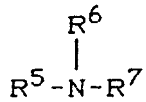

(IX)

in welchen

| | |
|---|---|
| Me | für ein- oder zweiwertige Metallionen |
| s und t | für die Zahl 1 und 2 und |
| $R^5$, $R^6$ und $R^7$ | unabhängig voneinander für Wasserstoff und Alkyl |

stehen,

gegebenenfalls in Gegenwart eines Verdünnungsmittels, umsetzt,

(E) zum Erhalt von Verbindungen der Formel (Ie)

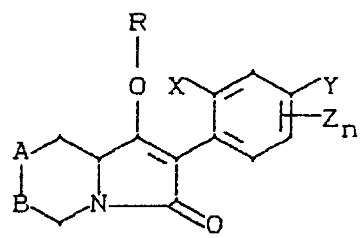

(Ie)

in welcher

A, B, R, X, Y, Z und n die oben angegebene Bedeutung haben,

Verbindungen der Formel (Ia)-(Ic),

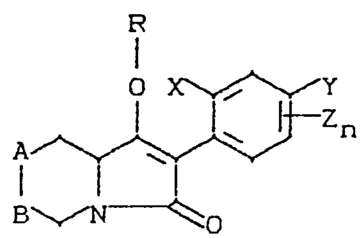

(Ia)-(Ic)

in welcher

R, X, Y, Z und n die oben angegebene Bedeutung haben

und

A-B für $-S-CH_2-$, $-CH_2-S-$ oder -S- steht,

mit annähernd äquimolaren Mengen eines Oxidationsmittels, gegebenenfalls in Gegenwart eines Verdünnungsmittels, umsetzt,

(F) zum Erhalt von
Verbindungen der Formel (If),

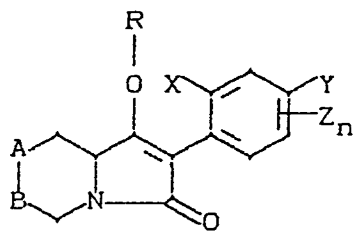

(If)

in welcher
A, B, R, X, Y, Z und n die oben angegebene Bedeutung haben,
Verbindungen der Formel (Ia)-(Ic),

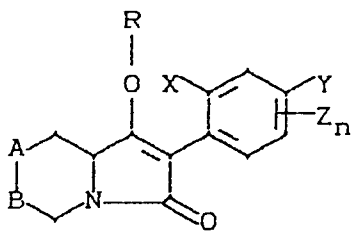

(Ia)-(Ic)

in welcher
R, X, Y, Z und n die oben angegebene Bedeutung haben
und
A-B für -S-$CH_2$-, -$CH_2$-S- oder -S-
mit doppelt äquimolaren Mengen eines Oxidationsmittels, gegebenenfalls in Gegenwart eines Verdünnungsmittels, umsetzt.

5. Insektizide und/oder akarizide und/oder herbizide Mittel, gekennzeichnet durch einen Gehalt an mindestens einem 3-Aryl-pyrrolidin-2,4-dion-Derivat der Formel (I).

6. Verfahren zur Bekämpfung von Insekten und/oder Spinnentieren und/oder Unkräutern, dadurch gekennzeichnet, daß man 3-Aryl-pyrrolidin-2,4-dion-Derivate der Formel (I) auf Insekten und/oder Spinnentiere und/oder Unkräutern und/oder deren Lebensraum einwirken läßt.

7. Verwendung von 3-Aryl-pyrrolidin-2,4-dion-Derivaten der Formel (I) zur Bekämpfung von Insekten und/oder Spinnentieren und/oder Unkräutern.

8. Verfahren zur Herstellung von insektiziden und/oder akariziden und/oder herbiziden Mitteln, dadurch gekennzeichnet, daß man 3-Aryl-pyrrolidin-2,4-dion-Derivate der Formel (I) mit Streckmitteln und/oder oberflächenaktiven Mitteln vermischt.

9. Acylaminosäureester der Formel (II),

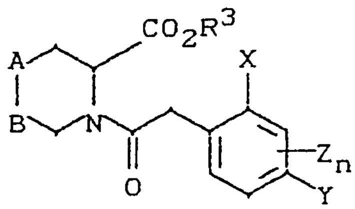

(II)

in welcher

A-B für $-S-CH_2-$, $-SO-CH_2-$, $-SO_2CH_2-$, $-CH_2-S-$, $-CH_2SO-$, $-CH_2SO_2-$, -S-, -SO-, $-SO_2-$ steht,
X für Alkyl, Halogen, Alkoxy steht,
Y für Wasserstoff, Alkyl, Halogen, Alkoxy, Halogenalkyl steht,
Z für Alkyl, Halogen, Alkoxy steht,
n für eine Zahl von 0-3 steht,
$R^3$ für Alkyl

steht.

**10.** Verfahren zur Herstellung von Acylaminosäureestern der Formel (II),

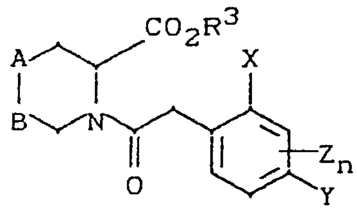

(II)

in welcher

A-B für $-S-CH_2-$, $-SO-CH_2-$, $-SO_2CH_2-$, $-CH_2-S-$, $-CH_2SO-$, $-CH_2SO_2-$, -S-, -SO-, $-SO_2-$ steht,
X für Alkyl, Halogen, Alkoxy steht,
Y für Wasserstoff, Alkyl, Halogen, Alkoxy, Halogenalkyl steht,
Z für Alkyl, Halogen, Alkoxy steht,
n für eine Zahl von 0-3 steht,
$R^3$ für Alkyl

steht, dadurch gekennzeichnet, daß man

a) Aminosäureester der Formel (VI),

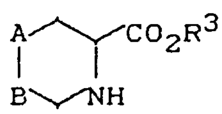

(VI)

in welcher

$R^3$ für Alkyl steht

und

A und B die oben angegebene Bedeutung haben,

mit Phenylessigsäurehalogeniden der Formel (VII)

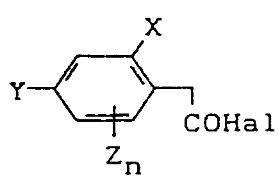

(VII)

in welcher

X, Y, Z und n die oben angegebene Bedeutung haben und
Hal für Chlor oder Brom steht,

acyliert

oder daß man

b) Acylaminosäuren der Formel (IIa),

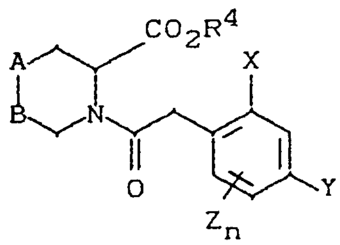

(IIa)

in welcher

A, B, X, Y, Z und n die oben angegebene Bedeutung haben

und

$R^4$ für Wasserstoff steht,

verestert.

## Claims

**1.** 3-Aryl-pyrrolidine-2,4-dione derivatives of the formula (I)

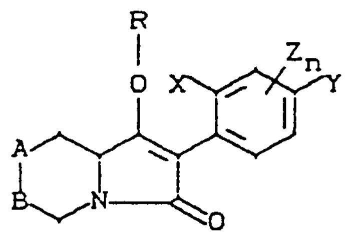

(I)

in which

A-B represents $-S-CH_2-$, $-SO-CH_2-$, $-SO_2CH_2-$, $-CH_2-S-$, $-CH_2SO-$, $-CH_2SO_2-$, -S-, -SO- or $-SO_2-$,

X represents $C_1$-$C_6$-alkyl, halogen or $C_1$-$C_6$-alkoxy,

Y represents hydrogen, $C_1$-$C_6$-alkyl, halogen, $C_1$-$C_6$-alkoxy or $C_1$-$C_3$-halogenoalkyl,

Z represents $C_1$-$C_6$-alkyl, halogen or $C_1$-$C_6$-alkoxy,

n represents a number from 0-3,

R represents hydrogen (Ia) or the groups of the formula

$-CO-R^1$ (Ib), $-CO-O-R^2$ (Ic)

or E (Id)

in which

E represents a metal cation equivalent or an ammonium ion,

$R^1$ represents optionally halogen-substituted $C_1$-$C_{20}$-alkyl, $C_2$-$C_{20}$-alkenyl, $C_1$-$C_8$-alkoxy-$C_1$-$C_8$-alkyl, $C_1$-$C_8$-alkylthio-$C_1$-$C_8$-alkyl, $C_1$-$C_8$-polyalkoxy-$C_2$-$C_8$-alkyl and cycloalkyl having 3-8 ring atoms, which may be interrupted by oxygen and/or sulphur,

represents phenyl which is optionally substituted by halogen, nitro, $C_1$-$C_6$-alkyl, $C_1$-$C_6$-alkoxy, $C_1$-$C_6$-halogenoalkyl or $C_1$-$C_6$-halogenoalkoxy;

represents phenyl-$C_1$-$C_6$-alkyl which is optionally substituted by halogen, $C_1$-$C_6$-alkyl, $C_1$-$C_6$-alkoxy, $C_1$-$C_6$-halogenoalkyl or $C_1$-$C_6$-halogenoalkoxy,

represents pyridyl, pyrimidyl, thiazolyl and pyrazolyl which are optionally substituted by halogen and $C_1$-$C_6$-alkyl,

represents phenoxy-$C_1$-$C_6$-alkyl optionally substituted by halogen and $C_1$-$C_6$-alkyl,

represents pyridyloxy-$C_1$-$C_4$-alkyl, pyrimidyloxy-$C_1$-$C_4$-alkyl and thiazolyloxy-$C_1$-$C_4$-alkyl which are optionally substituted by halogen, amino and $C_1$-$C_6$-alkyl,

$R^2$ represents $C_1$-$C_{20}$-alkyl, $C_2$-$C_{20}$-alkenyl, $C_1$-$C_8$-alkoxy-$C_2$-$C_8$-alkyl or $C_1$-$C_8$-polyalkoxy-$C_2$-$C_8$-alkyl which are optionally substituted by halogen,
represents phenyl optionally substituted by halogen, nitro, $C_1$-$C_6$-alkyl, $C_1$-$C_6$-alkoxy or $C_1$-$C_6$-halogenoalkyl,

and the enantiomerically pure forms of compounds of the formula (I).

**2.** 3-Aryl-pyrrolidine-2,4-2,4-diones of the formula (I) according to Claim 1, in which

A-B represents $-S-CH_2-$, $-SO-CH_2-$, $-SO_2-CH_2-$, $CH_2-S-$, $-CH_2-SO-$, $-CH_2-SO_2-$, -S-, -SO- or $-SO_2-$ ,
X represents $C_1$-$C_4$-alkyl, halogen or $C_1$-$C_4$-alkoxy,
Y represents hydrogen, $C_1$-$C_6$-alkyl, halogen, $C_1$-$C_4$-alkoxy or $C_1$-$C_2$-halogenoalkyl,
Z represents $C_1$-$C_4$-alkyl, halogen or $C_1$-$C_4$-alkoxy,
n represents a number from 0-3,
R represents hydrogen (Ia) or the groups of the formula

$-CO-R^1$ (Ib), $-CO-O-R^2$ (Ic)

or E (Id)

in which

E represents a metal cation equivalent or an ammonium ion,
$R^1$ represents optionally halogen-substituted $C_1$-$C_{16}$-alkyl, $C_2$-$C_{16}$-alkenyl, $C_1$-$C_6$-alkoxy-$C_1$-$C_6$-alkyl, $C_1$-$C_6$-alkylthio-$C_1$-$C_6$-alkyl, $C_1$-$C_6$-polyalkoxy-$C_2$-$C_6$-alkyl and cycloalkyl having 3-7 ring atoms, which can be interrupted by 1-2 oxygen and/or sulphur atoms,
represents phenyl optionally substituted by halogen, nitro, $C_1$-$C_4$-alkyl, $C_1$-$C_4$-alkoxy, $C_1$-$C_3$-halogenoalkyl or $C_1$-$C_3$-halogenoalkoxy,
represents phenyl-$C_1$-$C_4$-alkyl optionally substituted by halogen, $C_1$-$C_4$-alkyl, $C_1$-$C_4$-alkoxy, $C_1$-$C_3$-halogenoalkyl or $C_1$-$C_3$-halogenoalkoxy,
represents pyridyl, pyrimidyl, thiazolyl and pyrazolyl which are optionally substituted by halogen and $C_1$-$C_6$-alkyl,
represents phenoxy-$C_1$-$C_5$-alkyl optionally substituted by halogen and $C_1$-$C_4$-alkyl,
represents pyridyloxy-$C_1$-$C_4$-alkyl, pyrimidyloxy-$C_1$-$C_4$-alkyl and thiazolyloxy-$C_1$-$C_4$-alkyl which are optionally substituted by halogen, amino and $C_1$-$C_4$-alkyl,
$R^2$ represents $C_1$-$C_{16}$-alkyl, $C_2$-$C_{16}$-alkenyl, $C_1$-$C_{16}$-alkoxy-$C_2$-$C_6$-alkyl or $C_1$-$C_6$-polyalkoxy-$C_2$-$C_6$-alkyl which are optionally substituted by halogen, represents phenyl optionally substituted by halogen, nitro, $C_1$-$C_4$-alkyl, $C_1$-$C_3$-alkoxy or $C_1$-$C_3$-halogenoalkyl,

and the enantiomerically pure forms of compounds of the formula (I).

**3.** 3-Aryl-pyrrolidine-2,4-diones of the formula (I) according to Claim 1, in which

A-B represents $-S-CH_2-$, $-SO-CH_2-$, $-SO_2-CH_2-$, $-CH_2-S-$, $-CH_2-SO-$, $-CH_2SO_2-$, -S-, -SO- or $-SO_2$,
X represents methyl, ethyl, propyl, i-propyl, fluorine, chlorine, bromine, methoxy and ethoxy,
Y represents hydrogen, methyl, ethyl, propyl, i-propyl, butyl, i-butyl, tert.-butyl, chlorine, bromine, methoxy, ethoxy and trifluoromethyl,
Z represents methyl, ethyl, i-propyl, butyl, i-butyl, tert.-butyl, chlorine, bromine, methoxy and ethoxy,
n represents a number from 0-3,
R represents hydrogen (Ia) or the groups of the formula

$-CO-R^1$ (Ib), $-CO-O-R^2$ (Ic)

or E (Id)

in which

E represents a metal cation equivalent or an ammonium ion,
$R^1$ represents optionally flourine- or chlorine- substituted $C_1$-$C_{14}$-alkyl, $C_2$-$C_{14}$-alkenyl, $C_1$-$C_4$-alkoxy-$C_1$-$C_6$-alkyl, $C_1$-$C_4$-alkylthio-$C_1$-$C_6$-alkyl, $C_1$-$C_4$-polyalkoxy-$C_2$-$C_4$-alkyl and cycloalkyl having 3-6 ring atoms, which can be interrupted by 1-2 oxygen and/or sulphur atoms,
represents phenyl optionally substituted by fluorine, chlorine, bromine, methyl, ethyl, propyl,

i-propyl, methoxy, ethoxy, trifluoromethyl, trifluoromethoxy or nitro,
represents phenyl-$C_1$-$C_3$-alkyl optionally substituted by fluorine, chlorine, bromine, methyl, ethyl, propyl, i-propyl, methoxy, ethoxy, trifluoromethyl or trifluoromethoxy,
represents pyridyl, pyrimidyl, thiazolyl and pyrazolyl which are optionally substituted by fluorine, chlorine, bromine, methyl or ethyl,
represents phenoxy-$C_1$-$C_4$-alkyl optionally substituted by fluorine, chlorine, methyl or ethyl,
represents pyridyloxy-$C_1$-$C_4$-alkyl, pyrimidyloxy-$C_1$-$C_4$-alkyl and thiazolyloxy-$C_1$-$C_4$-alkyl which are optionally substituted by fluorine, chlorine, amino, methyl or ethyl,

$R^2$ represents $C_1$-$C_{14}$-alkyl, $C_2$-$C_{14}$-alkenyl, $C_1$-$C_4$-alkoxy-$C_2$-$C_6$-alkyl or $C_1$-$C_4$-polyalkoxy-$C_2$-$C_6$-alkyl which are optionally substituted by fluorine or chlorine,
represents phenyl optionally substituted by fluorine, chlorine, nitro, methyl, ethyl, propyl, i-propyl, methoxy, ethoxy or trifluoromethyl,

and the enantiomerically pure forms of compounds of the formula I.

**4.** Process for the preparation of 3-aryl-pyrrolidine-2,4-dione derivatives of the formula (I)

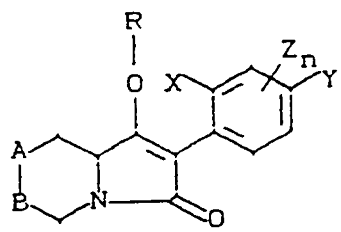

(I)

in which

A, B, R, X, Y, Z and n have the meaning given in Claim 1,

and the enantiomerically pure forms of compounds of the formula (I), characterized in that

(A) to obtain the compounds of the formula (Ia)

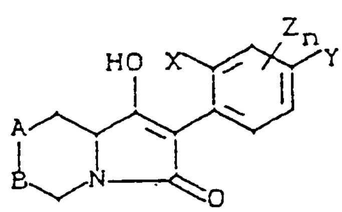

(Ia),

in which

A, B, X, Y, Z and n have the abovementioned meaning,

N-acylamino acid esters of the formula (II)

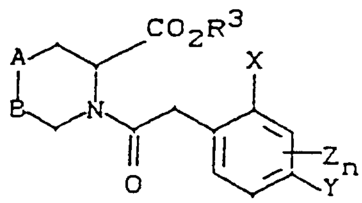

(II)

in which

A, B, X, Y, Z and n have the abovementioned meaning

and

$R^3$ represents alkyl,

are intramolecularly condensed in the presence of a diluent and in the presence of a base,

(B) to obtain compounds of the formula (Ib)

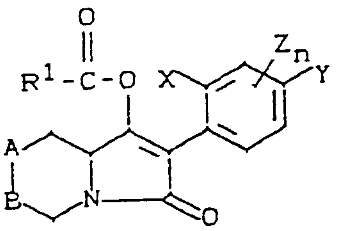

(Ib),

in which

A, B, X, Y, Z and n have the abovementioned meaning,

compounds of the formula (Ia)

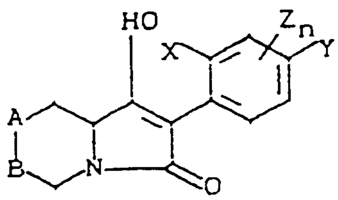

(Ia)

in which

A, B, X, Y, Z and n have the abovementioned meaning,

α) are reacted with acid halides of the general formula (III)

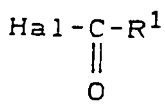

(III)

in which

$R^1$ has the abovementioned meaning

and

Hal represents halogen, in particular chlorine and bromine,

if appropriate in the presence of a diluent and if appropriate in the presence of an acid-binding agent,

or

β) are reacted with carboxylic acid anhydrides of the general formula (IV)

$R^1$-CO-O-CO-$R^1$ (IV)

in which

$R^1$ has the abovementioned meaning,

if appropriate in the presence of a diluent and if appropriate in the presence of an acid-binding agent,

(C) to obtain compounds of the formula (Ic)

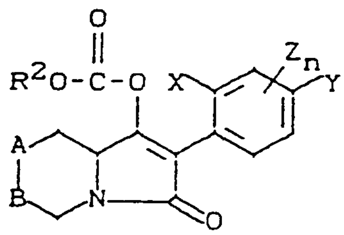

(Ic),

in which

A, B, X, Y, Z and n have the abovementioned meaning,

compounds of the formula (Ia)

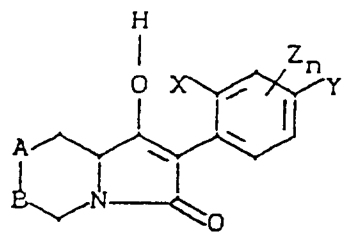

(Ia)

in which

A, B, X, Y, Z and n have the abovementioned meaning,

are reacted with chloroformic acid esters of the general formula (V)

$R^2$-O-CO-Cl (V)

in which

$R^2$ has the abovementioned meaning,

if appropriate in the presence of a diluent and if appropriate in the presence of an acid-binding agent,

(D) to obtain compounds of the formula (Id)

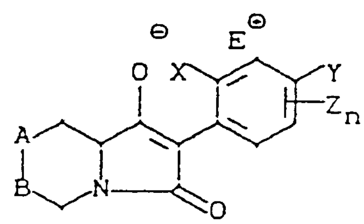

(Id)

in which

A, B, X, Y, Z, E and n have the abovementioned meaning,

compounds of the formula (Ia)

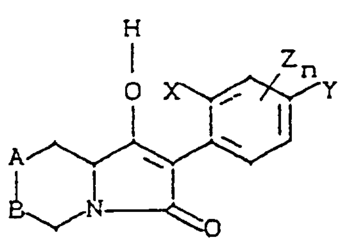

(Ia)

in which

A, B, X, Y, Z and n have the abovementioned meaning,

are reacted with metal hydroxides or amines of the general formulae (VIII) and (IX)

$Me_sOH_t$ (VIII)

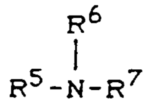 (IX)

in which

Me represents mono- or divalent metal ions,

s and t represent the number 1 and 2 and

$R^5$, $R^6$ and $R^7$ independently of one another represent hydrogen and alkyl,

if appropriate in the presence of a diluent,

(E) to obtain compounds of the formula (Ie)

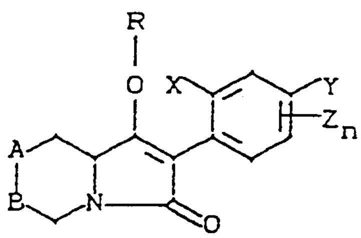 (Ie)

in which

A, B, R, X, Y, Z and n have the abovementioned meaning,

compounds of the formula (Ia)-(Ic)

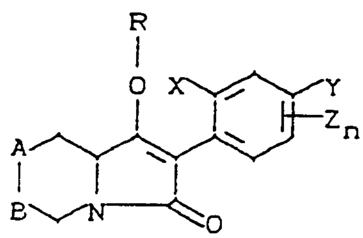 (Ia)-(Ic)

in which

R, X, Y, Z and n have the abovementioned meaning

and

A-B represents $-S-CH_2-$, $-CH_2-S-$ or -S-,

are reacted with approximately equimolar amounts of an oxidizing agent, if appropriate in the presence of a diluent,

(F) to obtain

compounds of the formula (If)

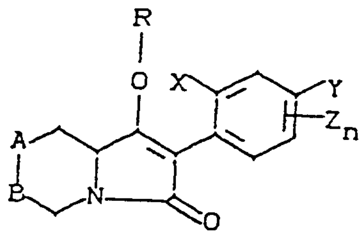

(If)

in which

A, B, R, X, Y, Z and n have the abovementioned meaning,

compounds of the formula (Ia)-(Ic)

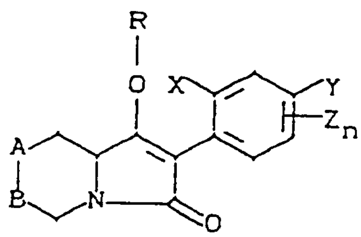

(Ia)-(Ic)

in which

R, X, Y, Z and n have the abovementioned meaning

and

A-B represents $-S-CH_2-$, $-CH_2-S-$ or -S-,

are reacted with double the equimolar amounts of an oxidizing agent, if appropriate in the presence of a diluent.

**5.** Insecticidal and/or acaricidal and/or herbicidal agents, characterized in that they contain at least one 3-aryl-pyrrolidine-2,4-dione derivative of the formula (I).

**6.** Method for combating insects and/or arachnida and/or weeds, characterized in that 3-aryl-pyrrolidine-2,4-dione derivatives of the formula (I) are allowed to act on insects and/or arachnida and/or weeds and/or their environment.

**7.** Use of 3-aryl-pyrrolidine-2,4-dione derivatives of the formula (I) for combating insects and/or arachnida and/or weeds.

**8.** Method for the production of insecticidal and/or acaricidal and/or herbicidal agents, characterized in that 3-aryl-pyrrolidine-2,4-dione derivatives of the formula (I) are mixed with extenders and/or surface-active agents.

**9.** Acylamino acid esters of the formula (II)

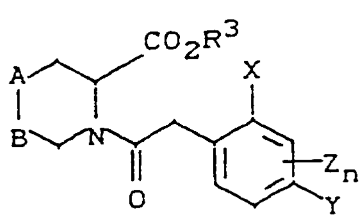

(II)

in which

A-B represents $-S-CH_2-$, $-SO-CH_2-$, $-SO_2CH_2-$, $-CH_2-S-$, $-CH_2SO-$, $-CH_2SO_2-$, -S-, -SO- or $-SO_2-$,

X represents alkyl, halogen or alkoxy,

Y represents hydrogen, alkyl, halogen, alkoxy or halogenoalkyl,
Z represents alkyl, halogen or alkoxy,
n represents a number from 0-3 and
$R^3$ represents alkyl.

**10.** Process for the preparation of acylamino acid esters of the formula (II)

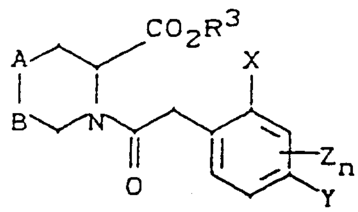

(II)

in which

A-B represents $-S-CH_2-$, $-SO-CH_2-$, $-SO_2CH_2-$, $-CH_2-S-$, $-CH_2SO-$, $-CH_2SO_2-$, $-S-$, $-SO-$ or $-SO_2-$,
X represents alkyl, halogen or alkoxy,
Y represents hydrogen, alkyl, halogen, alkoxy or halogenoalkyl,
Z represents alkyl, halogen or alkoxy,
n represents a number from 0-3 and
$R^3$ represents alkyl,

characterized in that

a) amino acid esters of the formula (VI)

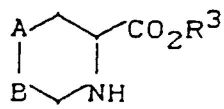

(VI)

in which
$R^3$ represents alkyl
and
A and B have the abovementioned meaning,
are acylated with phenylacetic acid halides of the formula (VII)

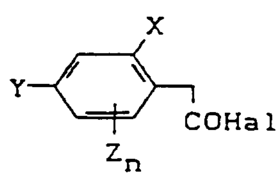

(VII)

in which
X, Y, Z and n have the abovementioned meaning and
Hal represents chlorine or bromine,
or in that

b) acylamino acids of the formula (IIa)

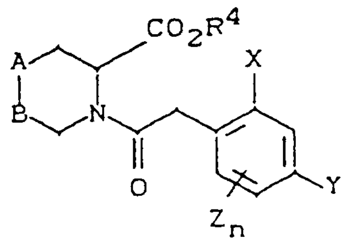

(IIa)

in which

A, B, X, Y, Z and n have the abovementioned meaning

and

$R^4$ represents hydrogen,

are esterified.

**Revendications**

**1.** 3-arylpyrrolidine-2,4-diones de formule (I)

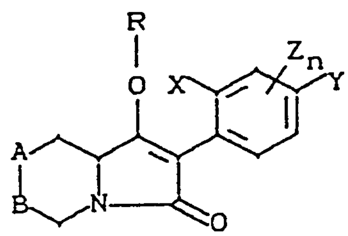

(I)

dans laquelle

A-B représentent $-S-CH_2-$, $-SO-CH_2-$, $-SO_2-CH_2-$, $-CH_2-S-$, $-CH_2-SO-$, $-CH_2-SO_2-$, -S-, -SO-, $-SO_2-$,

X est un groupe alkyle en $C_1$ à $C_6$, un halogène, un groupe alkoxy en $C_1$ à $C_6$,

Y représente de l'hydrogène, un groupe alkyle en $C_1$ à $C_6$, un halogène, un groupe alkoxy en $C_1$ à $C_6$, halogénalkyle en $C_1$ à $C_3$,

Z est un groupe alkyle en $C_1$ à $C_6$, un halogène, un groupe alkoxy en $C_1$ à $C_6$,

n est un nombre de 0 à 3,

R est de l'hydrogène (Ia) ou représente les groupes de formule

$-CO-R^1$ (Ib), $-CO-O-R^2$ (Ic)

ou E (Id)

dans lesquels

E est un équivalent de cation métallique ou un ion ammonium,

$R^1$ est un groupe alkyle en $C_1$ à $C_{20}$ éventuellement substitué par un halogène, alcényle en $C_2$ à $C_{20}$, (alkoxy en $C_1$ à $C_8$)-(alkyle en $C_1$ à $C_8$), (alkylthio en $C_1$ à $C_8$)-(alkyle en $C_1$ à $C_8$)-poly-(alkoxy en $C_1$ à $C_8$)-(alkyle en $C_2$ à $C_8$) et cycloalkyle à noyau de 3 à 8 atomes, qui peut être interrompu par de l'oxygène et/ou du soufre,

un groupe phényle éventuellement substitué par un halogène, un radical nitro, alkyle en $C_1$ à $C_6$, alkoxy en $C_1$ à $C_6$, halogénalkyle en $C_1$ à $C_6$, halogénalkoxy en $C_1$ à $C_6$,

un groupe phényl-(alkyle en $C_1$ à $C_6$) éventuellement substitué par un halogène, un radical alkyle en $C_1$ à $C_6$, alkoxy en $C_1$ à $C_6$, halogénalkyle en $C_1$ à $C_6$, halogénalkoxy en $C_1$ à $C_6$,

un groupe pyridyle, pyrimidyle, thiazolyle ou pyrazolyle substitués chacun le cas échéant par un halogène et un radical alkyle en $C_1$ à $C_6$, un groupe phénoxy-(alkyle en $C_1$ à $C_6$)

éventuellement substitué par un halogène et un radical alkyle en $C_1$ à $C_6$,
un groupe pyridyloxy-(alkyle en $C_1$ à $C_4$), pyrimidyloxy-(alkyle en $C_1$ à $C_4$) ou thiazolyloxy-(alkyle en $C_1$ à $C_4$) substitués chacun le cas échéant par un halogène, un radical amino et alkyle en $C_1$ à $C_6$

$R^2$ est un groupe alkyle en $C_1$ à $C_{20}$ éventuellemeent substitué par un halogène, alcényle en $C_2$ à $C_{20}$, (alkoxy en $C_1$ à $C_8$)-(alkyle en $C_2$ à $C_8$), poly(alkoxy en $C_1$ à $C_8$)-(alkyle en $C_2$ à $C_8$), un groupe phényle éventuellement substitué par un radical halogéno, nitro, alkyle en $C_1$ à $C_6$, alkoxy en $C_1$ à $C_6$, halogénalkyle en $C_1$ à $C_6$,

ainsi que les formes de pureté énantiomérique de composés de formule I.

**2.** 3-arylpyrrolidine-2,4-diones de formule (I) suivant la revendication 1, dans laquelle

A-B représentent $-S-CH_2-$, $-SO-CH_2-$, $-SO_2-CH_2-$, $-CH_2-S-$, $-CH_2-SO-$, $-CH_2-SO_2-$, -S-, -SO-, $-SO_2-$,

X est un groupe alkyle en $C_1$ à $C_4$, un halogène, un groupe alkoxy en $C_1$ à $C_4$,

Y est de l'hydrogène, un groupe alkyle en $C_1$ à $C_4$, un halogène, un groupe alkoxy en $C_1$ à $C_4$, halogénalkyle en $C_1$ ou $C_2$,

Z est un groupe alkyle en $C_1$ à $C_4$, un halogène, un groupe alkoxy en $C_1$ à $C_4$,

n est un nombre de 0 à 3,

R est de l'hydrogène (Ia) ou représente les groupes de formule

$-CO-R^1$ (Ib), $-CO-O-R^2$ (Ic)

ou E (Id)

dans lesquels

E est un équivalent de cation métallique ou un ion ammonium,

$R^1$ est un groupe alkyle en $C_1$ à $C_{16}$ éventuellement substitué par un halogène, alcényle en $C_2$ à $C_{16}$, (alkoxy en $C_1$ à $C_6$)-(alkyle en $C_1$ à $C_6$), (alkylthio en $C_1$ à $C_6$)-(alkyle en $C_1$ à $C_6$), poly-(alkoxy en $C_1$ à $C_6$)-(alkyle en $C_2$ à $C_6$) et cycloalkyle à noyau de 3 à 7 atomes qui peut être interrompu par 1 ou 2 atomes d'oxygène et/ou de soufre,
un groupe phényle éventuellement substitué par un radical halogéno, nitro, alkyle en $C_1$ à $C_4$, alkoxy en $C_1$ à $C_4$, halogénalkyle en $C_1$ à $C_3$, halogénalkoxy en $C_1$ à $C_3$,
un groupe phényl-(alkyle en $C_1$ à $C_4$) éventuellement substitué par un radical halogéno, alkyle en $C_1$ à $C_4$, alkoxy en $C_1$ à $C_4$, halogénalkyle en $C_1$ à $C_3$, halogénalkoxy en $C_1$ à $C_3$,
un groupe pyridyle, pyrimidyle, thiazolyle ou pyrazolyle éventuellement substitués chacun par un halogène et un radical alkyle en $C_1$ à $C_6$, un groupe phénoxy-(alkyle en $C_1$ à $C_5$) éventuellement substitué par un halogène et un radical alkyle en $C_1$ à $C_4$,
un groupe pyridyloxy-(alkyle en $C_1$ à $C_4$), pyrimidyloxy-(alkyle en $C_1$ à $C_4$) et thiazolyloxy-(alkyle en $C_1$ à $C_4$) substitués chacun le cas échéant par un halogène, un radical amino et alkyle en $C_1$ à $C_4$,

$R^2$ est un groupe alkyle en $C_1$ à $C_{16}$ éventuellemeent substitué par un halogène, alcényle en $C_2$ à $C_{16}$, (alkoxy en $C_1$ à $C_{16}$)-(alkyle en $C_2$ à $C_6$), poly(alkoxy en $C_1$ à $C_6$)-(alkyle en $C_2$ à $C_6$), un groupe phényle éventuellement substitué par un halogène, un radical nitro, alkyle en $C_1$ à $C_4$, alkoxy en $C_1$ à $C_3$, halogénalkyle en $C_1$ à $C_3$,

ainsi que les formes de pureté énantiomérique de composés de formule I.

**3.** 3-arylpyrrolidine-2,4-diones de formule (I) suivant la revendication 1, dans laquelle

A-B représentent $-S-CH_2-$, $-SO-CH_2-$, $-SO_2-CH_2-$, $-CH_2-S-$, $-CH_2-SO-$, $-CH_2SO_2-$, -S-, -SO-, $-SO_2-$,

X représente les radicaux méthyle, éthyle, propyle, isopropyle, fluoro, chloro, bromo, méthoxy et éthoxy,

Y représente de l'hydrogène, les radicaux méthyle, éthyle, propyle, isopropyle, butyle, isobutyle, tertiobutyle, chloro, bromo, méthoxy, éthoxy et trifluorométhyle,

Z représente les radicaux méthyle, éthyle, isopropyle, butyle, isobutyle, tertiobutyle, chloro, bromo, méthoxy et éthoxy,

n est un nombre de 0 à 3,

R représente de l'hydrogène (Ia) ou les groupes de formule

-CO-$R^1$ (Ib), -CO-O-$R^2$ (Ic)

ou E (Id)

dans lesquels

E est un équivalent de cation métallique ou un ion ammonium,

$R^1$ est un groupe alkyle en $C_1$ à $C_{14}$ éventuellement substitué par du fluor ou du chlore, un groupe alcényle en $C_2$ à $C_{14}$, (alkoxy en $C_1$ à $C_4$)-(alkyle en $C_1$ à $C_6$), (alkylthio en $C_1$ à $C_4$)-(alkyle en $C_1$ à $C_6$), poly(alkoxy en $C_1$ à $C_4$)-(alkyle en $C_2$ à $C_4$) et cycloalkyle à noyau de 3 à 6 atomes, qui peut être interrompu par 1-2 atomes d'oxygène et/ou de soufre, un groupe phényle portant éventuellement un substituant fluoro, chloro, bromo, méthyle, éthyle, propyle, isopropyle, méthoxy, éthoxy, trifluorométhyle, trifluorométhoxy, nitro,
un groupe phényl-(alkyle en $C_1$ à $C_3$) éventuellement substitué par un radical fluoro, chloro, bromo, méthyle, éthyle, propyle, isopropyle, méthoxy, éthoxy, trifluorométhyle, trifluorométhoxy,
un groupe pyridyle, pyrimidyle, thiazolyle ou pyrazolyle portant le cas échéant un substituant fluoro, chloro, bromo, méthyle, éthyle,
un groupe phénoxy-(alkyle en $C_1$ à $C_4$) éventuellement substitué par un radical fluoro, chloro, méthyle, éthyle,
un groupe pyridyloxy-(alkyle en $C_1$ à $C_4$), pyrimidyloxy-(alkyle en $C_1$ à $C_4$) ou thiazolyloxy-(alkyle en $C_1$ à $C_4$) portant éventuellement un substituant fluoro, chloro, amino, méthyle, éthyle,

$R^2$ est un groupe alkyle en $C_1$ à $C_{14}$ éventuellemeent substitué par du fluor ou du chlore, alcényle en $C_2$ à $C_{14}$, (alkoxy en $C_1$ à $C_4$)-(alkyle en $C_2$ à $C_6$), poly(alkoxy en $C_1$ à $C_4$)-(alkyle en $C_2$ à $C_6$),
un groupe phényle éventuellement substitué par un radical fluoro, chloro, nitro, méthyle, éthyle, propyle, isopropyle, méthoxy, éthoxy, trifluorométhyle,

ainsi que les formes de pureté énantiomérique de composés de formule I.

**4.** Procédé de production de 3-arylpyrrolidine-2,4-diones de formule (I)

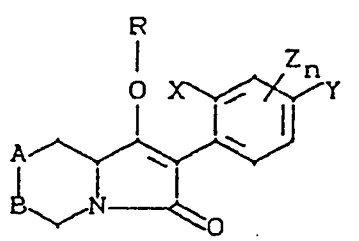

(I)

dans laquelle

A, B, R, X, Y, Z et n ont les définitions indiquées dans la revendication 1 ainsi que des formes de pureté énantiomérique de composés de formule (I), caractérisé en ce que

(A) pour obtenir les composés de formule (Ia)

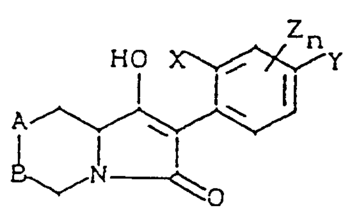

(Ia),

dans laquelle

A, B, X, Y, Z et n ont la définition indiquée ci-dessus,

on effectue la condensation intramoléculaire d'esters de N-acylamino-acides de formule (II)

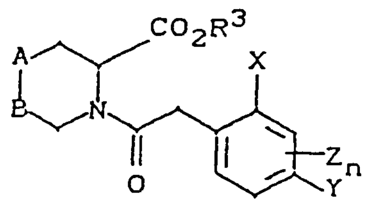

(II)

dans laquelle

A, B, X, Y, Z et n ont la définition indiquée ci-dessus, et

$R^3$ est un groupe alkyle

en présence d'un diluant et en présence d'une base,

(B) pour l'obtention de composés de formule (Ib)

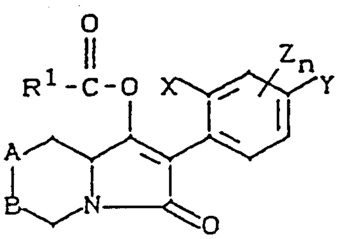

(Ib),

dans laquelle

A, B, X, Y, Z et n ont la définition indiquée ci-dessus,

on fait réagir des composés de formule (Ia)

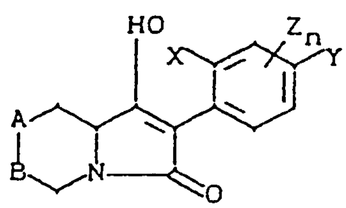

(Ia)

dans laquelle

A, B, X, Y, Z et n ont la définition indiquée ci-dessus,

α) avec des halogénures d'acides de formule générale (III),

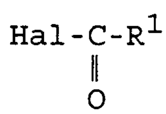

(III)

dans laquelle

$R^1$ a la définition indiquée ci-dessus et

Hal est un halogène, en particulier le chlore et le brome,

le cas échéant en présence d'un diluant et en la présence éventuelle d'un accepteur d'acide, ou

β) avec des anhydrides d'acides carboxyliques de formule (IV)

$R^1$-CO-O-CO-$R^1$ (IV)

dans laquelle

$R^1$ a la définition indiquée ci-dessus,

le cas échéant en présence d'un diluant et en la présence éventuellement d'un accepteur d'acide,

(C) pour obtenir des composés de formule (Ic)

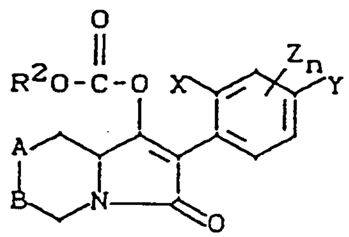

(Ic),

dans laquelle

A, B, X, Y, Z et n ont la définition indiquée ci-dessus,

on fait réagir des composés de formule (Ia)

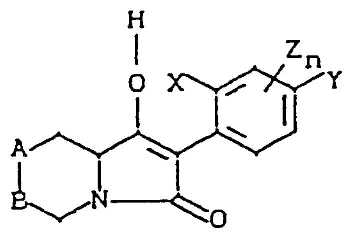

(Ia)

dans laquelle

A, B, X, Y, Z et n ont la définition indiquée ci-dessus,

avec un ester d'acide chloroformique de formule générale (V)

$R^2$-O-CO-Cl (V)

dans laquelle

$R^2$ a la définition indiquée ci-dessus,

le cas échéant en présence d'un diluant et en la présence éventuellement d'un accepteur d'acide,

(D) pour l'obtention de composés de formule (Id)

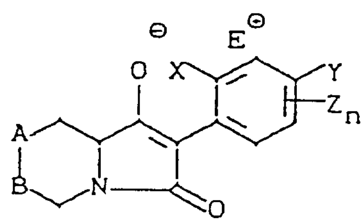

(Id)

dans laquelle

A, B, X, Y, Z, E et n ont la définition indiquée ci-dessus,

on fait réagir des composés de formule (Ia)

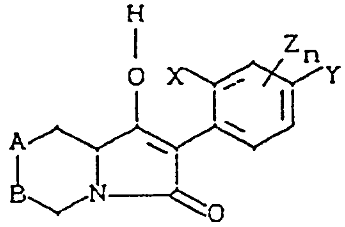

(Ia)

dans laquelle

A, B, X, Y, Z et n ont la définition indiquée ci-dessus,

avec des hydroxydes de métaux ou des amines de formules générales (VIII) et (IX)

$Me_sOH_t$ (VIII)

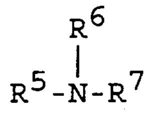 (IX)

dans lesquelles

Me représente des ions métalliques monovalents ou divalents

s et t représentent les nombres 1 et 2 et

$R^5$, $R^6$ et $R^7$ représentent indépendamment les uns des autres de l'hydrogène et des groupes alkyle,

le cas échéant en présence d'un diluant,

(E) pour l'obtention de composés de formule (Ie)

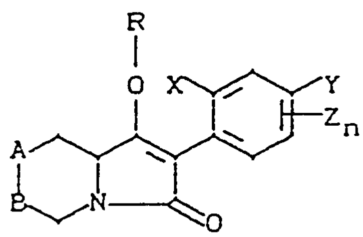 (Ie)

dans laquelle

A, B, R, X, Y, Z et n ont la définition indiquée ci-dessus,

on fait réagir des composés de formule (Ia)-(Ic),

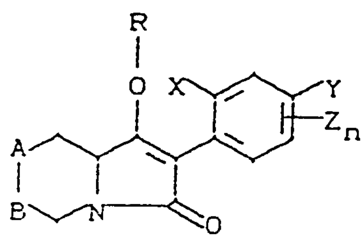 (Ia)-(Ic)

dans laquelle

R, X, Y, Z et n ont la définition indiquée ci-dessus, et

A-B représentent $-S-CH_2-$, $-CH_2-S-$ ou -S-,

avec des quantités à peu près équimolaires d'un agent oxydant, éventuellement en présence d'un diluant,

(F) pour l'obtention de composés de formule (If),

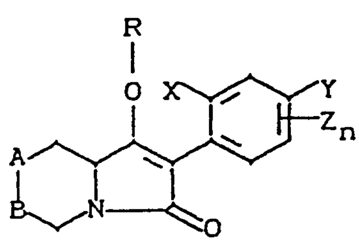 (If)

dans laquelle

A, B, R, X, Y, Z et n ont la définition indiquée ci-dessus,

on fait réagir des composés de formule (Ia)-(Ic),

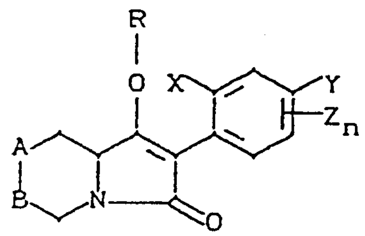

(Ia)-(Ic)

dans laquelle

R, X, Y, Z et n ont la définition indiquée ci-dessus, et

A-B représentent -S-$CH_2$-, -$CH_2$-S- ou -S-, avec le double des quantités équimolaires d'un

agent oxydant, le cas échéant en présence d'un diluant.

**5.** Compositions insecticides et/ou acaricides et/ou herbicides, caractérisées par une teneur en au moins une 3-arylpyrrolidine-2,4-dione de formule (I).

**6.** Procédé pour combattre des insectes et/ou des acariens et/ou des mauvaises herbes, caractérisé en ce qu'on fait agir sur les insectes et/ou sur les acariens et/ou sur les mauvaises herbes et/ou sur leur milieu des 3-arylpyrrolidine-2,4-diones de formule (I).

**7.** Utilisation de 3-arylpyrrolidine-2,4-diones de formule (I) pour combattre des insectes et/ou des acariens et/ou des mauvaises herbes.

**8.** Procédé de préparation de compositions insecticides et/ou acaricides et/ou herbicides, caractérisé en ce qu'on mélange des 3-arylpyrrolidine-2,4-diones de formule (I) avec des diluants et/ou des agents tensioactifs.

**9.** Esters d'acylamino-acides de formule (II)

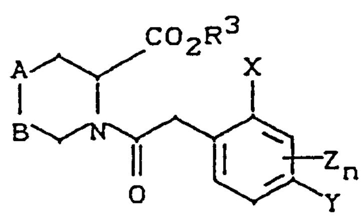

(II)

dans laquelle

A-B représentent -S-$CH_2$-, -SO-$CH_2$-, -$SO_2$-$CH_2$-, -$CH_2$-S-, -$CH_2$-SO-, -$CH_2SO_2$-, -S-, -SO-, -$SO_2$-,

X est un groupe alkyle, un halogène, un groupe alkoxy,

Y est de l'hydrogène, un groupe alkyle, un halogène, un groupe alkoxy, halogénalkyle,

Z est un groupe alkyle, un halogène, un groupe alkoxy,

n est un nombre de 0 à 3,

$R^3$ est un groupe alkyle.

**10.** Procédé de production d'esters d'acylamino-acides de formule (II)

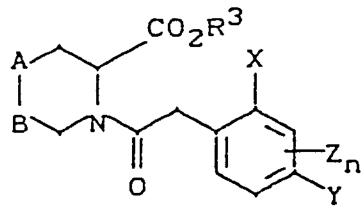

(II)

dans laquelle

A-B représentent -S-$CH_2$-, -SO-$CH_2$-, -$SO_2$-$CH_2$-, -$CH_2$-S-, -$CH_2$-SO-, -$CH_2SO_2$-, -S-, -SO-, -$SO_2$-,

X est un groupe alkyle, un halogène, un groupe alkoxy,

Y est de l'hydrogène, un groupe alkyle, un halogène, un groupe alkoxy, halogénalkyle,

Z est un groupe alkyle, un halogène, un groupe alkoxy,

n est un nombre de 0 à 3,

$R^3$ est un groupe alkyle,

caractérisé en ce que

a) on acyle des esters d'amino-acides de formule (VI)

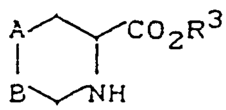

(VI)

dans laquelle

$R^3$ est un groupe alkyle,

et

A et B ont la définition indiquée ci-dessus,

avec des halogénures d'acides phénylacétiques de formule (VII)

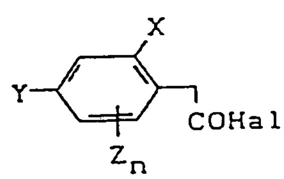

(VII)

dans laquelle

X, Y, Z et n ont la définition indiquée ci-dessus,

et

Hal représente du chlore ou du brome,

ou bien

b) on estérifie des acylamino-acides de formule (IIa)

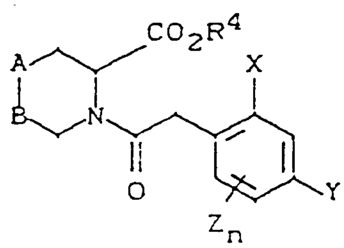

(IIa)

dans laquelle

A, B, X, Y, Z et n ont la définition indiquée ci-dessus
et
$R^4$ est de l'hydrogène.